# EUROPEAN PATENT APPLICATION

(11) **EP 4 772 652 A1**
(43) Date of publication of application: **08.07.2026**
(21) Application number: 24860409.2
(22) Date of filing: 28.08.2024
(51) Int. Cl.: C12Q 1/6895, C12N 5/04, A61K 8/9789, A61K 8/60, A61K 8/14, A61Q 19/00, A61Q 19/02, A61Q 19/08

(54) **BIOMARKER FOR VERIFYING PLANT CALLUS-DERIVED EXOSOMES AND METHOD FOR ISOLATING EXOSOMES USING SAME**

(30) Priority: 28.08.2023 KR 20230112479
(71) Applicant: GFC Life Science Co., Ltd., Hwaseong-si, Gyeonggi-do 18471 (KR)
(72) Inventor: KANG, Hee-Cheol, Hwaseong-si, Gyeonggi-do 18471 (KR); KIM, Mi-Jung, Seoul 05402 (KR); KIM, Jae-Goo, Hwaseong-si, Gyeonggi-do 18471 (KR); MIN, Jin-Woo, Hwaseong-si, Gyeonggi-do 18471 (KR); JIN, Soll, Hwaseong-si, Gyeonggi-do 18471 (KR); KANG, Hyeon-Tae, Hwaseong-si, Gyeonggi-do 18471 (KR)
(74) Representative: EIP
(86) International application number: PCT/KR2024/012855
(87) International publication number: WO 2025/048489

(57) **Abstract**

The present invention relates to: a biomarker for verifying plant callus-derived exosomes; a method for isolating plant callus-derived exosomes using same; a cosmetic composition; a pharmaceutical composition; and a filler.

## Description

### Technical Field

The present invention relates to a biomarker for validating exosomes derived from plant callus and a method of isolating exosomes using the same.

### Background Art

Exosomes are nano-sized extracellular vesicles with a diameter of about 200 nm or less that are formed in the cells of all living organisms and secreted extracellularly through multivesicular bodies of phospholipid bilayer membranes. All animal, plant, and microbial cells secrete exosomes, which are the smallest units of cells and contain various cell-derived proteins, nucleic acids (DNA, messenger RNA, or micro RNA, etc.), lipids, metabolites, etc. depending on their origin. Exosomes have the effect of promoting skin regeneration and alleviating skin inflammation, and stimulate the production of beneficial substances for the skin, such as collagen and hyaluronic acid. In particular, they have been proven to be effective in improving skin tone, increasing skin moisturization, enhancing elasticity through increased collagen synthesis, reducing fine wrinkles, and alleviating acne and atopic dermatitis by alleviating skin inflammation.

Despite these advantages, research on exosomes and their applied products still remain limited. One reason therefor is that methods capable of obtaining exosomes are still limited, and even these methods are capable of extracting only a very small amount of specific exosomes from total exosomes and have low extraction efficiency. Therefore, improving these methods is essential for research and product commercialization.

As attempts to use exosomes as next-generation drug delivery vehicles and therapeutic agents tend to increase continuously, the development of mass-production and high-purity exosome purification methods is required. While significant progress has been made in the development of kits for isolating small amounts of exosomes for diagnostic purposes, the development of cell lines for mass production, the development of processes, the development of technology for high-purity isolation of exosomes, and the development of exosome analysis technology for quality control still remain insufficient. Therefore, there is a need for research and securing of original technologies to develop high-purity exosome production, isolation, and quality control technologies.

Exosomes contain specific genetic materials and various bioactive factors, such as proteins, lipids, and secondary metabolites, depending on the nature and characteristics of the cell of origin. In particular, they contain large amounts of miRNAs, non-coding nucleotides. These miRNAs in exosomes have been suggested to have potential gene regulatory functions and potential uses as biomarkers.

miRNA is a single-stranded, non-coding small RNA, usually consisting of 19 to 25 nucleotides (nt). miRNA, first discovered in 1993 by Professor Victor Ambros' research group in the mechanism that controls the growth of *Caenorhabditis elegans* larvae, has been recognized as merely playing an auxiliary role that transfers the genetic information of DNA to messenger RNA (mRNA), which is then transcribed into transfer RNA (tRNA) by the ribosome in the cytoplasm, thereby synthesizing each encoded amino acid-based protein.

However, miRNAs have recently been attracting attention as key regulatory factors that play an important role in gene expression in most animal, plant, and viral species. With advances in biotechnology and the completion of the Human Genome Project, the development of agents for treating incurable diseases and cancer using gene expression regulation has been actively made. In particular, research is currently underway to use miRNAs, which play a crucial role in regulating gene expression, to diagnose various diseases. At least 60% of human mRNAs are regulated by miRNAs, which function as important *in vivo* regulators which are also associated with various cancers, including breast cancer and bladder cancer, as well as various diseases such as diabetes and dementia. Thus, research is actively underway to use, as biomarkers for diseases of interest, the increase or decrease of specific miRNAs in blood, urine, saliva, and cells with lesions.

Plants accounting for 82% of the Earth's biomass represent a correspondingly high diversity of species. Research on the efficacy of plant-derived exosomes is also actively underway across various plants, and these exosomes exhibit specific effects depending on the plant's unique characteristics. In particular, numerous miRNAs found in plants play a crucial role in the overall signaling network, from environmental stress signal recognition to the expression of stress-responsive genes. In addition, during plant growth and development, miRNAs are involved not only in the physiological aspects of flowering and growth regulation, but also in the morphological aspects of regulating the formation and shape of organs such as leaves and flowers.

In Korea, existing studies on exosome technology using plants include Korean Patent No. 10-2267796 (entitled "Composition for skin soothing containing exosomes derived from natural extracts as active ingredient") and Korean Patent No. 10-2523528 (entitled "Cosmetic composition containing exosomes derived from grapefruit and camellia flower as active ingredient"). As disclosed in these documents, exosomes are mainly identified only by whether their size is nano-sized or their morphology is a phospholipid bilayer.

Although exosomes have been actively studied in various aspects, including components, functions, isolation and analysis, diagnosis, and treatment methods, the distinction between exosomes, which are extracellular vesicles released by plant cells, and endosomes, which are intracellular vesicles existing within plant cells, remains ambiguous. In this regard, the development of biomarkers to confirm or validate exosomes is required, and as part thereof, research and analysis of miRNAs are required.

Accordingly, the present inventors have proposed specific miRNAs as biomarkers for validating plant callus-derived exosomes to determine whether isolated exosomes in the production of plant-derived exosomes are exosomes, which are extracellular vesicles released by plant cells, or endosomes, which are intracellular vesicles existing within plant cells. In addition, the present inventors have found that, by providing a method for isolating exosomes derived from plant callus, which includes a step of validating exosomes by identifying specific miRNAs as described above, exosomes can be clearly validated and thus quality competitiveness can be ensured, thereby completing the present invention.

### DISCLOSURE

### Technical Problem

An object of the present invention is to provide a biomarker for validating plant callus-derived exosomes.

Another object of the present invention is to provide a method for isolating plant callus-derived exosomes containing specific miRNA.

Still another object of the present invention is to provide a cosmetic, pharmaceutical or filler composition for skin improvement containing, as an active ingredient, plant callus-derived exosomes containing specific miRNA.

### Technical Solution

To achieve the above objects, the present invention provides a biomarker for validating plant callus-derived exosomes, which is selected from the group consisting of miR156 of SEQ ID NO: 1, miR156b of SEQ ID NO: 2, miR157 of SEQ ID NO: 3, miR159 of SEQ ID NO: 4, miR162 of SEQ ID NO: 5, miR166 of SEQ ID NO: 6, miR167 of SEQ ID NO: 7, miR168 of SEQ ID NO: 8, miR169 of SEQ ID NO: 9, miR171 of SEQ ID NO: 10, miR172 of SEQ ID NO: 11, miR319 of SEQ ID NO: 12, miR393 of SEQ ID NO: 13, miR395 of SEQ ID NO: 14, miR396 of SEQ ID NO: 15, miR397 of SEQ ID NO: 16, miR398 of SEQ ID NO: 17, miR408 of SEQ ID NO: 18, miR477 of SEQ ID NO: 19, miR482a of SEQ ID NO: 20, miR482b of SEQ ID NO: 21, miR827 of SEQ ID NO: 22, miR1311 of SEQ ID NO: 23, miR1511 of SEQ ID NO: 24, miR1515 of SEQ ID NO: 25, miR3701 of SEQ ID NO: 26, miR4407 of SEQ ID NO: 27, miR4415 of SEQ ID NO: 28, miR6024 of SEQ ID NO: 29, miR6300 of SEQ ID NO: 30, gfc-lal-miR001 of SEQ ID NO: 31, gfc-lal-miR002 of SEQ ID NO: 32, gfc-lal-miR003 of SEQ ID NO: 33, gfc-lal-miR004 of SEQ ID NO: 34, gfc-lal-miR005 of SEQ ID NO: 35, gfc-cas-miR001 of SEQ ID NO: 36, gfc-cas-miR002 of SEQ ID NO: 37, gfc-cas-miR003 of SEQ ID NO: 38, gfc-cas-miR004 of SEQ ID NO: 39, and gfc-pgi-miR001 of SEQ ID NO: 40.

The present invention also provides a method for isolating plant callus-derived exosomes, including: a step of isolating exosomes from plant callus; a step of extracting small RNA from the exosomes isolated from plant callus; a step of analyzing the nucleotide sequence of the extracted small RNA to obtain sequence data of miRNA; and a step of validating the isolated exosomes by identifying at least one miRNA selected from the group consisting of miR156 of SEQ ID NO: 1, miR156b of SEQ ID NO: 2, miR157 of SEQ ID NO: 3, miR159 of SEQ ID NO: 4, miR162 of SEQ ID NO: 5, miR166 of SEQ ID NO: 6, miR167 of SEQ ID NO: 7, miR168 of SEQ ID NO: 8, miR169 of SEQ ID NO: 9, miR171 of SEQ ID NO: 10, miR172 of SEQ ID NO: 11, miR319 of SEQ ID NO: 12, miR393 of SEQ ID NO: 13, miR395 of SEQ ID NO: 14, miR396 of SEQ ID NO: 15, miR397 of SEQ ID NO: 16, miR398 of SEQ ID NO: 17, miR408 of SEQ ID NO: 18, miR477 of SEQ ID NO: 19, miR482a of SEQ ID NO: 20, miR482b of SEQ ID NO: 21, miR827 of SEQ ID NO: 22, miR1311 of SEQ ID NO: 23, miR1511 of SEQ ID NO: 24, miR1515 of SEQ ID NO: 25, miR3701 of SEQ ID NO: 26, miR4407 of SEQ ID NO: 27, miR4415 of SEQ ID NO: 28, miR6024 of SEQ ID NO: 29, miR6300 of SEQ ID NO: 30, gfc-lal-miR001 of SEQ ID NO: 31, gfc-lal-miR002 of SEQ ID NO: 32, gfc-lal-miR003 of SEQ ID NO: 33, gfc-lal-miR004 of SEQ ID NO: 34, gfc-lal-miR005 of SEQ ID NO: 35, gfc-cas-miR001 of SEQ ID NO: 36, gfc-cas-miR002 of SEQ ID NO: 37, gfc-cas-miR003 of SEQ ID NO: 38, gfc-cas-miR004 of SEQ ID NO: 39, and gfc-pgi-miR001 of SEQ ID NO: 40, which is expressed in the exosomes isolated from plant callus, from the nucleotide sequence of the miRNA.

In the present invention, the step of isolating the exosomes from plant callus may include a step of culturing the plant callus in a liquid suspension, separating the culture medium from the callus culture by filtration, subjecting the culture medium to low-frequency ultrasound and centrifugation to obtain a supernatant extract, and subjecting the obtained supernatant extract to a three-step purification process including tangential flow filtration (TFF), ultracentrifugation (UC), and bind-elute size-exclusion chromatography (BE-SEC), thereby isolating exosomes.

In the present invention, the plant may be at least one selected from the group consisting of *Asteraceae, Apiaceae, Rutaceae, Rosaceae, Vitaceae, Brassicaceae, Selaginellaceae, Oleaceae, Nelumbonaceae, Araliaceae, Lamiaceae, Hypericaceae, Pedaliaceae, Ginkgoaceae,* and *Piperaceae.*

In the present invention, the plant may be at least one selected from the group consisting of *Leontopodium alpinum, Centella asiatica,* and *Panax ginseng.*

In the present invention, the plant may be at least one terrestrial plant selected from the group consisting of dicotyledons, monocotyledons, and ferns.

In the present invention, the analysis of the nucleotide sequence may be performed by measurement using a next-generation sequencing (NGS) method.

The present invention also provides a plant callus-derived exosome obtained by the isolation method.

In the present invention, the exosome may be loaded with at least one active ingredient for a cosmetic, pharmaceutical or filler composition for skin improvement, which is selected from vitamins, growth factors, hormones, sugar molecules, nucleic acids, lipids, and peptides.

The present invention also provides a cosmetic composition for skin improvement containing, as an active ingredient, plant callus-derived exosomes containing at least one miRNA selected from the group consisting of miR156 of SEQ ID NO: 1, miR156b of SEQ ID NO: 2, miR157 of SEQ ID NO: 3, miR159 of SEQ ID NO: 4, miR162 of SEQ ID NO: 5, miR166 of SEQ ID NO: 6, miR167 of SEQ ID NO: 7, miR168 of SEQ ID NO: 8, miR169 of SEQ ID NO: 9, miR171 of SEQ ID NO: 10, miR172 of SEQ ID NO: 11, miR319 of SEQ ID NO: 12, miR393 of SEQ ID NO: 13, miR395 of SEQ ID NO: 14, miR396 of SEQ ID NO: 15, miR397 of SEQ ID NO: 16, miR398 of SEQ ID NO: 17, miR408 of SEQ ID NO: 18, miR477 of SEQ ID NO: 19, miR482a of SEQ ID NO: 20, miR482b of SEQ ID NO: 21, miR827 of SEQ ID NO: 22, miR1311 of SEQ ID NO: 23, miR1511 of SEQ ID NO: 24, miR1515 of SEQ ID NO: 25, miR3701 of SEQ ID NO: 26, miR4407 of SEQ ID NO: 27, miR4415 of SEQ ID NO: 28, miR6024 of SEQ ID NO: 29, miR6300 of SEQ ID NO: 30, gfc-lal-miR001 of SEQ ID NO: 31, gfc-lal-miR002 of SEQ ID NO: 32, gfc-lal-miR003 of SEQ ID NO: 33, gfc-lal-miR004 of SEQ ID NO: 34, gfc-lal-miR005 of SEQ ID NO: 35, gfc-cas-miR001 of SEQ ID NO: 36, gfc-cas-miR002 of SEQ ID NO: 37, gfc-cas-miR003 of SEQ ID NO: 38, gfc-cas-miR004 of SEQ ID NO: 39, and gfc-pgi-miR001 of SEQ ID NO: 40.

In the present invention, the skin improvement may be at least one selected from the group consisting of skin moisturizing, whitening, elasticity, regeneration, wrinkle reduction, anti-aging, skin soothing, and anti-inflammation.

The present invention also provides a pharmaceutical composition for skin improvement containing, as an active ingredient, plant callus-derived exosomes containing at least one miRNA selected from the group consisting of miR156 of SEQ ID NO: 1, miR156b of SEQ ID NO: 2, miR157 of SEQ ID NO: 3, miR159 of SEQ ID NO: 4, miR162 of SEQ ID NO: 5, miR166 of SEQ ID NO: 6, miR167 of SEQ ID NO: 7, miR168 of SEQ ID NO: 8, miR169 of SEQ ID NO: 9, miR171 of SEQ ID NO: 10, miR172 of SEQ ID NO: 11, miR319 of SEQ ID NO: 12, miR393 of SEQ ID NO: 13, miR395 of SEQ ID NO: 14, miR396 of SEQ ID NO: 15, miR397 of SEQ ID NO: 16, miR398 of SEQ ID NO: 17, miR408 of SEQ ID NO: 18, miR477 of SEQ ID NO: 19, miR482a of SEQ ID NO: 20, miR482b of SEQ ID NO: 21, miR827 of SEQ ID NO: 22, miR1311 of SEQ ID NO: 23, miR1511 of SEQ ID NO: 24, miR1515 of SEQ ID NO: 25, miR3701 of SEQ ID NO: 26, miR4407 of SEQ ID NO: 27, miR4415 of SEQ ID NO: 28, miR6024 of SEQ ID NO: 29, miR6300 of SEQ ID NO: 30, gfc-lal-miR001 of SEQ ID NO: 31, gfc-lal-miR002 of SEQ ID NO: 32, gfc-lal-miR003 of SEQ ID NO: 33, gfc-lal-miR004 of SEQ ID NO: 34, gfc-lal-miR005 of SEQ ID NO: 35, gfc-cas-miR001 of SEQ ID NO: 36, gfc-cas-miR002 of SEQ ID NO: 37, gfc-cas-miR003 of SEQ ID NO: 38, gfc-cas-miR004 of SEQ ID NO: 39, and gfc-pgi-miR001 of SEQ ID NO: 40.

The present invention also provides a filler composition for skin improvement containing, as an active ingredient, plant callus-derived exosomes containing at least one miRNA selected from the group consisting of miR156 of SEQ ID NO: 1, miR156b of SEQ ID NO: 2, miR157 of SEQ ID NO: 3, miR159 of SEQ ID NO: 4, miR162 of SEQ ID NO: 5, miR166 of SEQ ID NO: 6, miR167 of SEQ ID NO: 7, miR168 of SEQ ID NO: 8, miR169 of SEQ ID NO: 9, miR171 of SEQ ID NO: 10, miR172 of SEQ ID NO: 11, miR319 of SEQ ID NO: 12, miR393 of SEQ ID NO: 13, miR395 of SEQ ID NO: 14, miR396 of SEQ ID NO: 15, miR397 of SEQ ID NO: 16, miR398 of SEQ ID NO: 17, miR408 of SEQ ID NO: 18, miR477 of SEQ ID NO: 19, miR482a of SEQ ID NO: 20, miR482b of SEQ ID NO: 21, miR827 of SEQ ID NO: 22, miR1311 of SEQ ID NO: 23, miR1511 of SEQ ID NO: 24, miR1515 of SEQ ID NO: 25, miR3701 of SEQ ID NO: 26, miR4407 of SEQ ID NO: 27, miR4415 of SEQ ID NO: 28, miR6024 of SEQ ID NO: 29, miR6300 of SEQ ID NO: 30, gfc-lal-miR001 of SEQ ID NO: 31, gfc-lal-miR002 of SEQ ID NO: 32, gfc-lal-miR003 of SEQ ID NO: 33, gfc-lal-miR004 of SEQ ID NO: 34, gfc-lal-miR005 of SEQ ID NO: 35, gfc-cas-miR001 of SEQ ID NO: 36, gfc-cas-miR002 of SEQ ID NO: 37, gfc-cas-miR003 of SEQ ID NO: 38, gfc-cas-miR004 of SEQ ID NO: 39, and gfc-pgi-miR001 of SEQ ID NO: 40.

### Advantageous Effects

According to the present invention, miR156 of SEQ ID NO: 1, miR156b of SEQ ID NO: 2, miR157 of SEQ ID NO: 3, miR159 of SEQ ID NO: 4, miR162 of SEQ ID NO: 5, miR166 of SEQ ID NO: 6, miR167 of SEQ ID NO: 7, miR168 of SEQ ID NO: 8, miR169 of SEQ ID NO: 9, miR171 of SEQ ID NO: 10, miR172 of SEQ ID NO: 11, miR319 of SEQ ID NO: 12, miR393 of SEQ ID NO: 13, miR395 of SEQ ID NO: 14, miR396 of SEQ ID NO: 15, miR397 of SEQ ID NO: 16, miR398 of SEQ ID NO: 17, miR408 of SEQ ID NO: 18, miR477 of SEQ ID NO: 19, miR482a of SEQ ID NO: 20, miR482b of SEQ ID NO: 21, miR827 of SEQ ID NO: 22, miR1311 of SEQ ID NO: 23, miR1511 of SEQ ID NO: 24, miR1515 of SEQ ID NO: 25, miR3701 of SEQ ID NO: 26, miR4407 of SEQ ID NO: 27, miR4415 of SEQ ID NO: 28, miR6024 of SEQ ID NO: 29, miR6300 of SEQ ID NO: 30, gfc-lal-miR001 of SEQ ID NO: 31, gfc-lal-miR002 of SEQ ID NO: 32, gfc-lal-miR003 of SEQ ID NO: 33, gfc-lal-miR004 of SEQ ID NO: 34, gfc-lal-miR005 of SEQ ID NO: 35, gfc-cas-miR001 of SEQ ID NO: 36, gfc-cas-miR002 of SEQ ID NO: 37, gfc-cas-miR003 of SEQ ID NO: 38, gfc-cas-miR004 of SEQ ID NO: 39, and gfc-pgi-miR001 of SEQ ID NO: 40 may be used as biomarkers for validating plant callus-derived exosomes by identifying that they are expressed in the plant callus-derived exosomes.

In addition, by providing a method for isolating plant callus-derived exosomes containing specific miRNA, it is possible to ensure quality competitiveness by clearly proving that the isolated exosomes are exosomes.

In addition, plant callus-derived exosomes containing specific miRNA may be used in various fields, including cosmetics and pharmaceuticals related to skin improvement purposes.

### Brief Description of Drawings

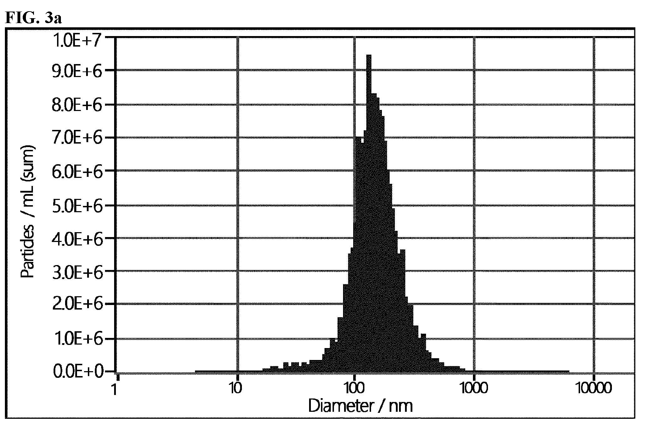

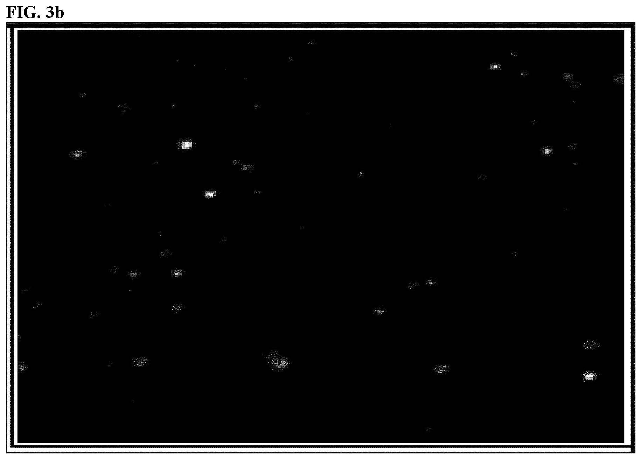

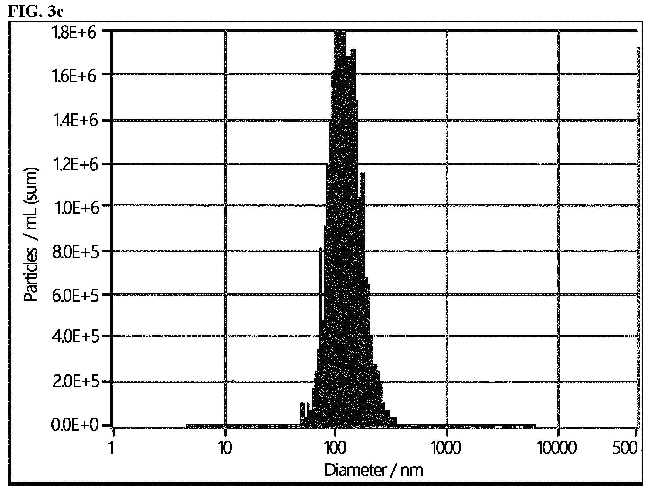

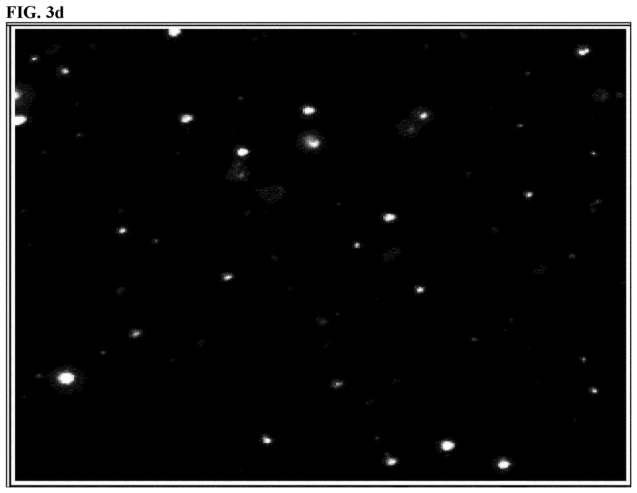

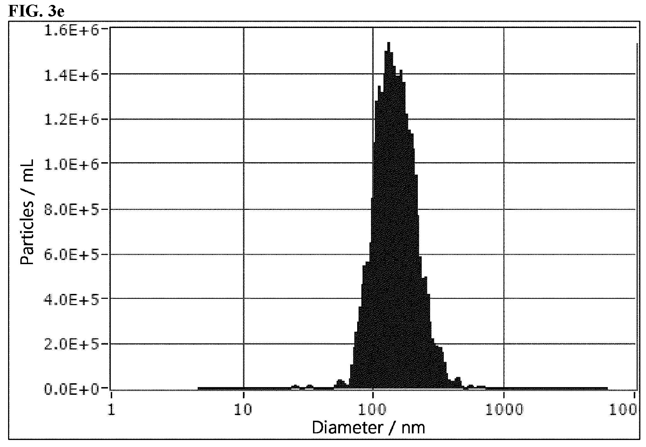

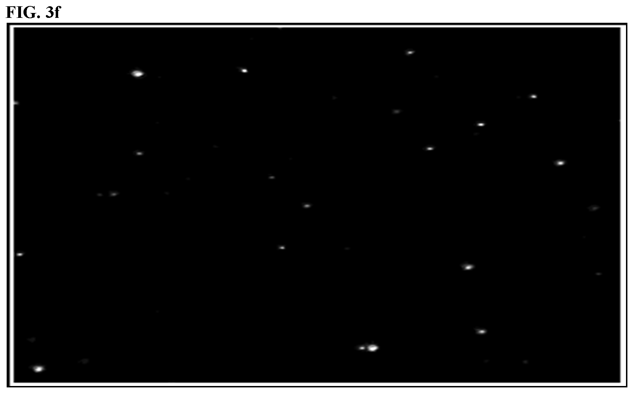

FIG. 1 is a schematic diagram showing a method for isolating plant callus-derived exosomes.
FIG. 2 is a schematic diagram showing next-generation sequencing (NGS) performed to identify miRNAs in plant callus and plant callus-derived exosomes.
FIGS. 3a to 3f show the results of analyzing *Leontopodium alpinum* callus-derived exosomes, *Centella asiatica* callus-derived exosomes, and *Panax ginseng* callus-derived exosomes using a nanoparticle tracking analyzer.
Specifically, FIGS. 3a and 3b show a particle size distribution graph and microscopic observation photograph of *Leontopodium alpinum* stem exosomes, respectively, as a result of NTA analysis of *Leontopodium alpinum* callus according to an example of the present invention. FIGS. 3c and 3d show a particle size distribution graph and microscopic observation photograph of *Panax ginseng* stem exosomes, respectively, as a result of NTA analysis of *Panax ginseng* callus according to an example of the present invention. FIGS. 3e and 3f show a particle size distribution graph and microscopic observation photograph of *Centella asiatica* stem exosomes, respectively, as a result of NTA analysis of *Centella asiatica* callus according to an example of the present invention.

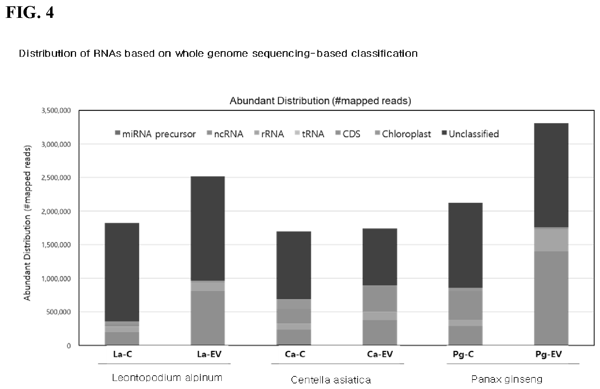

FIG. 4 is a graph showing the distribution of RNAs present in *Leontopodium alpinum* callus, *Centella asiatica* callus, and *Panax ginseng* callus, and in *Leontopodium alpinum* callus-derived exosomes, *Centella asiatica* callus-derived exosomes, and *Panax ginseng* callus-derived exosomes, as a result of NGS-based small RNA analysis.

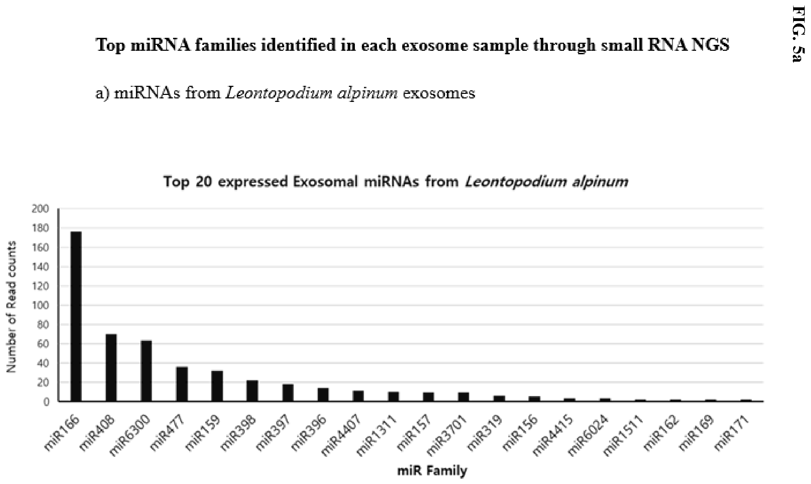

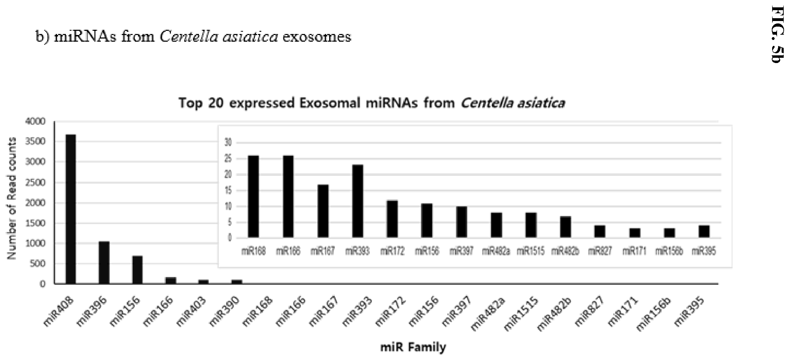

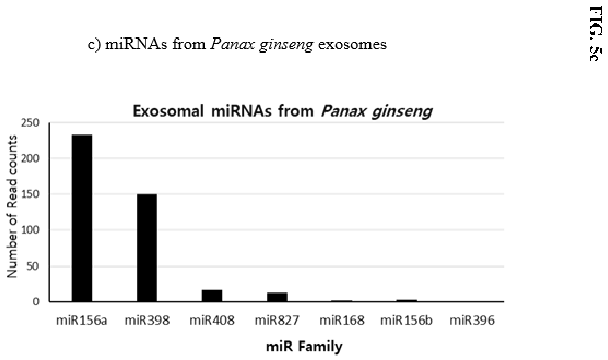

FIGS. 5a to 5c are graphs showing the top miRNA families in *Leontopodium alpinum* callus-derived exosomes, *Centella asiatica* callus-derived exosomes, and *Panax ginseng* callus-derived exosomes, respectively, identified through NGS-based small RNA analysis.

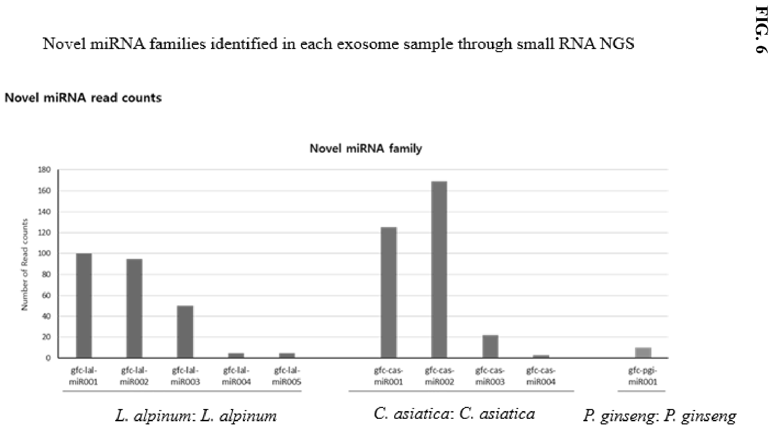

FIG. 6 is a graph showing novel miRNA families in *Leontopodium alpinum* callus-derived exosomes, *Centella asiatica* callus-derived exosomes, and *Panax ginseng* callus-derived exosomes identified through NGS-based small RNA analysis.

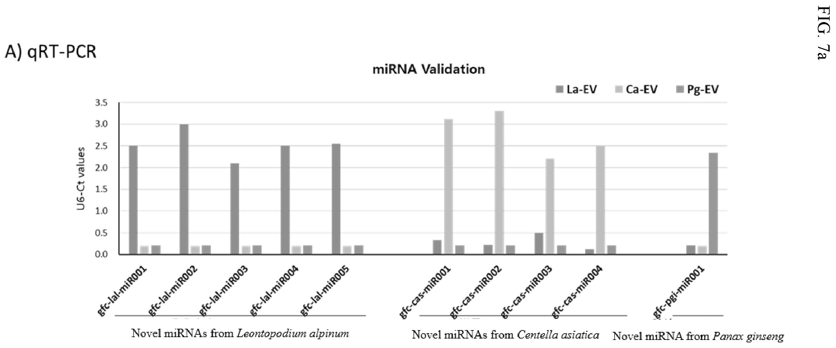

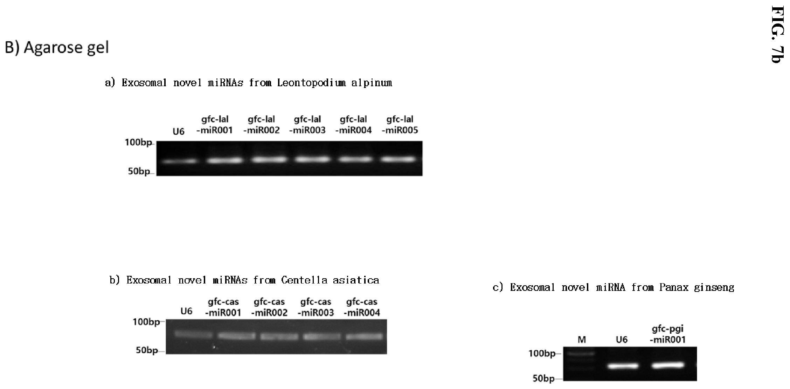

FIGS. 7a and 7b show the expression patterns of miRNAs in *Leontopodium alpinum* callus-derived exosomes, *Centella asiatica* callus-derived exosomes, and *Panax ginseng* callus-derived exosomes validated through qRT-PCR (FIG. 7a, A) and electrophoresis images of each PCR product (FIG. 7b, B).

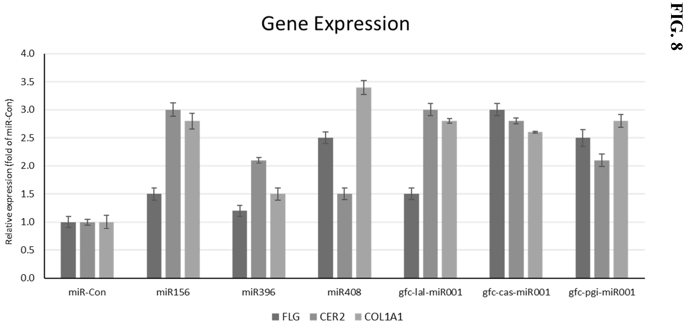

FIG. 8 is a graph showing the results of analyzing the efficacy of expression of FLG, COL1A1, and CER2 by miRNAs in plant callus-derived exosomes in skin cells.

### Best Mode

Unless otherwise defined, all technical and scientific terms used in the present specification have the same meanings as commonly understood by those skilled in the art to which the present disclosure pertains. In general, the nomenclature used in the present specification is well known and commonly used in the art.

Throughout the present specification, when any part is referred to as "including" any component, this means that the part does not exclude other components, but may include other components, unless otherwise specifically stated.

In the present invention, the term "exosomes" refers to nano-sized vesicles with a diameter of about 200 nm or less that are formed in the cells of all living organisms and secreted extracellularly through multivesicular bodies of phospholipid bilayer membranes, and is used interchangeably with the term "extracellular vesicles".

### <Biomarkers for exosome validation, method for isolating exosomes, and exosomes>

The present invention relates to biomarkers for exosome validation, a method for isolating exosomes, and exosomes, and specifically, encompasses micro RNAs (miRNAs) as biomarkers capable of validating plant callus-derived exosomes, a method of isolating exosomes from plant callus using the same, and exosomes isolated thereby.

The biomarker for exosome validation according to the present invention may be one or a combination of two or more selected from the group consisting of miR156 of SEQ ID NO: 1, miR156b of SEQ ID NO: 2, miR157 of SEQ ID NO: 3, miR159 of SEQ ID NO: 4, miR162 of SEQ ID NO: 5, miR166 of SEQ ID NO: 6, miR167 of SEQ ID NO: 7, miR168 of SEQ ID NO: 8, miR169 of SEQ ID NO: 9, miR171 of SEQ ID NO: 10, miR172 of SEQ ID NO: 11, miR319 of SEQ ID NO: 12, miR393 of SEQ ID NO: 13, miR395 of SEQ ID NO: 14, miR396 of SEQ ID NO: 15, miR397 of SEQ ID NO: 16, miR398 of SEQ ID NO: 17, miR408 of SEQ ID NO: 18, miR477 of SEQ ID NO: 19, miR482a of SEQ ID NO: 20, miR482b of SEQ ID NO: 21, miR827 of SEQ ID NO: 22, miR1311 of SEQ ID NO: 23, miR1511 of SEQ ID NO: 24, miR1515 of SEQ ID NO: 25, miR3701 of SEQ ID NO: 26, miR4407 of SEQ ID NO: 27, miR4415 of SEQ ID NO: 28, miR6024 of SEQ ID NO: 29, miR6300 of SEQ ID NO: 30, gfc-lal-miR001 of SEQ ID NO: 31, gfc-lal-miR002 of SEQ ID NO: 32, gfc-lal-miR003 of SEQ ID NO: 33, gfc-lal-miR004 of SEQ ID NO: 34, gfc-lal-miR005 of SEQ ID NO: 35, gfc-cas-miR001 of SEQ ID NO: 36, gfc-cas-miR002 of SEQ ID NO: 37, gfc-cas-miR003 of SEQ ID NO: 38, gfc-cas-miR004 of SEQ ID NO: 39, and gfc-pgi-miR001 of SEQ ID NO: 40.

The biomarker for exosome validation according to the present invention may include at least one selected from SEQ ID NO: 1, SEQ ID NO: 15, SEQ ID NO: 18, SEQ ID NO: 31, SEQ ID NO: 36, and SEQ ID NO: 40. More preferably, the biomarker for exosome validation according to the present invention may include at least one selected from SEQ ID NO: 1, SEQ ID NO: 15, and SEQ ID NO: 18, and in this case, the biomarker may validate all or each of exosomes derived from *Leontopodium alpinum, Centella asiatica* and/or *Panax ginseng,* preferably callus of *Leontopodium alpinum, Centella asiatica* and/or *Panax ginseng.*

The miRNA molecules of SEQ ID NOs: 1 to 40 may each independently have a length of 10 to 50 nucleotides, preferably 17 to 23 nucleotides, without being limited thereto.

By confirming the presence or absence of the miRNA molecules of SEQ ID NOs: 1 to 40, it is possible to confirm whether plant callus-derived exosomes have been isolated. Specifically, by confirming whether at least of the above specific miRNAs is included, it is possible to clearly prove the presence of exosomes, which are extracellular vesicles released from plant cells. Thus the miRNAs of SEQ ID NOs: 1 to 40 may be used as biomarkers for validating plant callus-derived exosomes.

Moreover, the present invention relates to a method for isolating plant callus-derived exosomes, including: a step of isolating exosomes from plant callus; a step of extracting small RNA from the exosomes isolated from plant callus; a step of analyzing the nucleotide sequence of the extracted small RNA to obtain sequence data of miRNA; and a step of validating the isolated exosomes by identifying at least one miRNA selected from the group consisting of miR156 of SEQ ID NO: 1, miR156b of SEQ ID NO: 2, miR157 of SEQ ID NO: 3, miR159 of SEQ ID NO: 4, miR162 of SEQ ID NO: 5, miR166 of SEQ ID NO: 6, miR167 of SEQ ID NO: 7, miR168 of SEQ ID NO: 8, miR169 of SEQ ID NO: 9, miR171 of SEQ ID NO: 10, miR172 of SEQ ID NO: 11, miR319 of SEQ ID NO: 12, miR393 of SEQ ID NO: 13, miR395 of SEQ ID NO: 14, miR396 of SEQ ID NO: 15, miR397 of SEQ ID NO: 16, miR398 of SEQ ID NO: 17, miR408 of SEQ ID NO: 18, miR477 of SEQ ID NO: 19, miR482a of SEQ ID NO: 20, miR482b of SEQ ID NO: 21, miR827 of SEQ ID NO: 22, miR1311 of SEQ ID NO: 23, miR1511 of SEQ ID NO: 24, miR1515 of SEQ ID NO: 25, miR3701 of SEQ ID NO: 26, miR4407 of SEQ ID NO: 27, miR4415 of SEQ ID NO: 28, miR6024 of SEQ ID NO: 29, miR6300 of SEQ ID NO: 30, gfc-lal-miR001 of SEQ ID NO: 31, gfc-lal-miR002 of SEQ ID NO: 32, gfc-lal-miR003 of SEQ ID NO: 33, gfc-lal-miR004 of SEQ ID NO: 34, gfc-lal-miR005 of SEQ ID NO: 35, gfc-cas-miR001 of SEQ ID NO: 36, gfc-cas-miR002 of SEQ ID NO: 37, gfc-cas-miR003 of SEQ ID NO: 38, gfc-cas-miR004 of SEQ ID NO: 39, and gfc-pgi-miR001 of SEQ ID NO: 40, which is expressed in the exosomes isolated from plant callus, from the nucleotide sequence of the miRNA.

According to one embodiment of the present invention, the isolation method of isolating the exosomes from plant callus may include a step of culturing the plant callus in a liquid suspension, separating the culture medium from the callus culture by filtration, subjecting the culture medium to low-frequency ultrasound and centrifugation to obtain a supernatant extract, and subjecting the obtained supernatant extract to a three-step purification process including tangential flow filtration (TFF), ultracentrifugation (UC), and bind-elute size-exclusion chromatography (BE-SEC), thereby isolating exosomes.

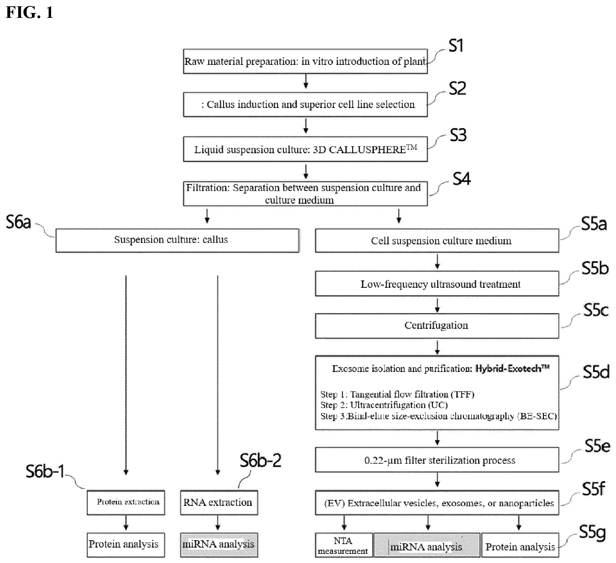

FIG. 1 is a schematic diagram showing in more detail a method of isolating exosomes derived from plant callus according to one embodiment of the present invention, but the scope of the present invention is not limited thereto. First, a plant introduced *in vitro* is prepared (S1). The plant introduced *in vitro* may be one introduced *in vitro* using plant cell tissue culture technology, without being limited thereto. Thereafter, callus is induced from each plant and a superior cell line is selected (S2). A step of creating a culture environment in which exosomes may be released from the callus into a culture medium for the selected superior cell line may be further included (S3). This step may be a step of creating a culture environment in which exosomes may be released from the callus into the culture medium, through liquid suspension culture (3D CALLUSPHERE^{™}) using an air-injected bioreactor, without being limited thereto. Liquid suspension culture (3D CALLUSPHERE^{™}) is a plant cell suspension culture technology. It is a sterile 3D callus culture system in which cells may be cultured smoothly in suspension without stagnation by injecting air at an appropriate flow rate through an air pump in a bioreactor, a cell culture vessel made of glass, and the gas accumulated in the culture vessel through respiration may be discharged through the exhaust port. Thereafter, a step of separating the suspension culture medium from the suspension callus culture may be further included (S4). In one example of the present invention, this step may be performed by filtration. Specifically, this step may be a step of separating the liquid suspension culture, which has been cultured for about 4 to 6 weeks while maintaining the supply amount of oxygen-containing air at 0.2 to 0.4 vvm using an air pump, into the callus culture and the culture medium through a 100- to 400-µm mesh sieve.

Hereinafter, the process of treating the separated suspension culture medium will be described in detail (**S5a**). Specifically, the suspension culture medium is subjected to physical stimulation of the cell membrane through low-frequency ultrasound treatment (**S5b**), and then centrifuged at 1,500 to 2,000xg for 20 to 30 minutes using a centrifuge (**S5c**), and then only the supernatant is collected, thereby separating and removing cell debris. The supernatant extract is then subjected to a Hybrid-Exotech^{™} process for pure exosome extraction and concentration (**S5d**). The Hybrid-Exotech^{™} process is performed by a three-step process. Here, the first step is tangential flow filtration (TFF) using a 300-kDa annular porous membrane filter. In this step, material is dispersed and circulated using continuous hydraulic pressure, thereby concentrating the material to about 5 to 10 times the initial starting volume based on particle size or molecular weight and performing first purification. In the second step, the exosomes concentrated and purified by TFF are subjected to second purification using ultracentrifugation at 100,000 to 150,000xg for 90 min. Finally, in the third step, third purification of collecting only pure nano-sized exosomes from which non-extracellular vesicles and large proteins have been removed is performed through bind-elute size-exclusion chromatography (BE-SEC) according to molecular size and molecular weight. The plant callus culture-derived exosomes obtained through the three-step Hybrid-Exotech^{™} process are filtered, for example, through a 0.22-µm filter to obtain the final plant callus-derived exosomes that have been subjected to the sterilization process (**S5e**). Meanwhile, the callus culture obtained through the culturing is subjected to RNA extraction and miRNA analysis for comparative analysis with exosome-derived miRNAs ().

The present invention may provide a method of isolating plant callus-derived exosomes through a step of analyzing miRNAs in the plant callus, preferably the plant callus-derived exosomes, most preferably the exosomes obtained from the cell suspension culture medium of the plant callus, and validating the exosomes by identifying whether the exosomes contain at least one selected from the group consisting of miR156 of SEQ ID NO: 1, miR156b of SEQ ID NO: 2, miR157 of SEQ ID NO: 3, miR159 of SEQ ID NO: 4, miR162 of SEQ ID NO: 5, miR166 of SEQ ID NO: 6, miR167 of SEQ ID NO: 7, miR168 of SEQ ID NO: 8, miR169 of SEQ ID NO: 9, miR171 of SEQ ID NO: 10, miR172 of SEQ ID NO: 11, miR319 of SEQ ID NO: 12, miR393 of SEQ ID NO: 13, miR395 of SEQ ID NO: 14, miR396 of SEQ ID NO: 15, miR397 of SEQ ID NO: 16, miR398 of SEQ ID NO: 17, miR408 of SEQ ID NO: 18, miR477 of SEQ ID NO: 19, miR482a of SEQ ID NO: 20, miR482b of SEQ ID NO: 21, miR827 of SEQ ID NO: 22, miR1311 of SEQ ID NO: 23, miR1511 of SEQ ID NO: 24, miR1515 of SEQ ID NO: 25, miR3701 of SEQ ID NO: 26, miR4407 of SEQ ID NO: 27, miR4415 of SEQ ID NO: 28, miR6024 of SEQ ID NO: 29, miR6300 of SEQ ID NO: 30, gfc-lal-miR001 of SEQ ID NO: 31, gfc-lal-miR002 of SEQ ID NO: 32, gfc-lal-miR003 of SEQ ID NO: 33, gfc-lal-miR004 of SEQ ID NO: 34, gfc-lal-miR005 of SEQ ID NO: 35, gfc-cas-miR001 of SEQ ID NO: 36, gfc-cas-miR002 of SEQ ID NO: 37, gfc-cas-miR003 of SEQ ID NO: 38, gfc-cas-miR004 of SEQ ID NO: 39, and gfc-pgi-miR001 of SEQ ID NO: 40 among the expressed miRNAs.

According to one embodiment of the present invention, the plant may be at least one selected from the group consisting of *Asteraceae, Apiaceae, Rutaceae, Rosaceae, Vitaceae, Brassicaceae, Selaginellaceae, Oleaceae, Nelumbonaceae, Araliaceae, Lamiaceae, Hypericaceae, Pedaliaceae, Ginkgoaceae,* and *Piperaceae.*

In addition, the plant may be at least one terrestrial plant selected from the group consisting of dicotyledons, monocotyledons, and ferns. In one example of the present invention, the plant may be at least one selected from the group consisting of *Leontopodium alpinum, Centella asiatica,* and *Panax ginseng,* without being limited thereto.

According to one embodiment of the present invention, the analysis of the nucleotide sequence may be performed by measurement using a next-generation sequencing (NGS) method.

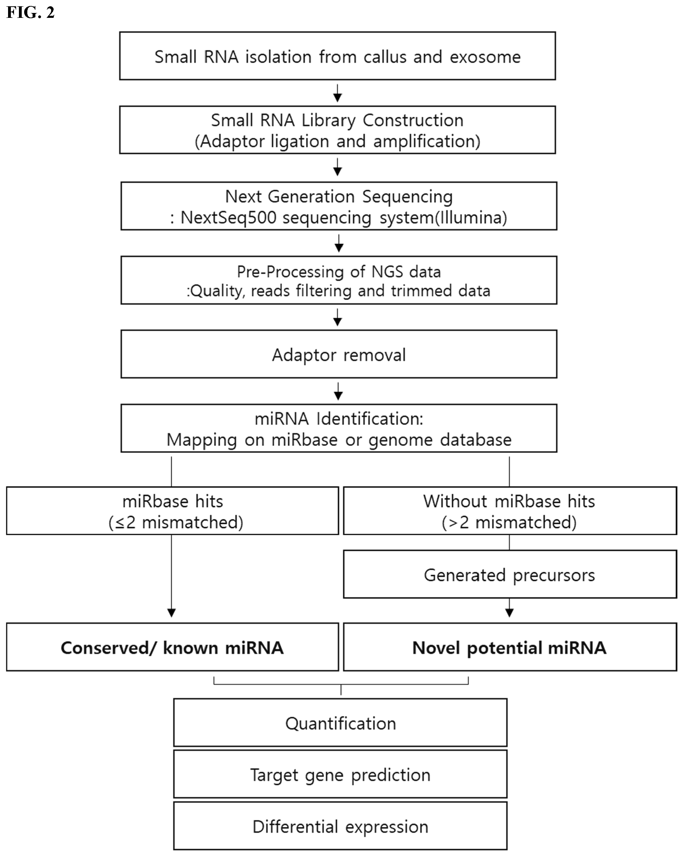

FIG. 2 is a schematic diagram showing a next-generation sequencing (NGS)-based small RNA sequence analysis method performed to analyze miRNAs in plant callus or plant callus-derived exosomes and a method for miRNA validation.

First, small RNA from plant callus or plant callus-derived exosomes was extracted. Small RNA extraction from a plant callus sample was performed using a XENO-Plant small RNA Purification kit (Xenohelix, Korea), and small RNA extraction from plant callus-derived exosomes was performed using a XENO-EVARI kit (Xenohelix, Korea). NGS library construction and analysis are performed for small RNA analysis. Using a XENO-Libera library kit (Xenohelix, Korea), 3' and 5' adapters were ligated to both ends, and cDNA was generated by reverse transcription, and then amplified. The constructed library was sequenced using the MiniSeq sequencing system (NextSeq500, Illumina). Raw data were checked for quality using FastQC v0.11. 9, and adapter sequences and low-quality 3'-end sequences were removed (quality score < 22) using Cutadapt v 2.8, and the read length was trimmed to 18 to 30 bases. The identified miRNAs were validated by calculating the number of aligned reads for each sample and the RPTM (Reads Per Ten Million) value. The miRNA candidates thus obtained were analyzed using the existing miRNA database, miRBase. If there were up to two mismatches in the sequence, the miRNA was designated as a previously reported conserved or known miRNA. If there were two or more mismatches, the miRNA was designated as an unreported novel potential miRNA. Each miRNA is validated through quantification, target gene prediction, and differential expression.

In the method of isolating plant callus-derived exosomes by identifying miRNA, it is possible to validate whether the isolated exosomes are exosomes, which are extracellular vesicles released by plant cells, or endosomes, which are intracellular vesicles existing in plant cells, and thus quality competitiveness is excellent.

In addition, the present invention relates to a plant callus-derived exosome obtained by the above-described isolation method.

In the present invention, the exosome may be loaded with at least one active ingredient for a cosmetic, pharmaceutical or filler composition for skin improvement, which is selected from vitamins, growth factors, hormones, sugar molecules, nucleic acids, lipids, and peptides.

### <Cosmetic composition, pharmaceutical composition and filler composition for skin improvement, and method using the same>

The present invention relates to a cosmetic composition for skin improvement containing, as an active ingredient, plant callus-derived exosomes containing at least one miRNA selected from the group consisting of miR156 of SEQ ID NO: 1, miR156b of SEQ ID NO: 2, miR157 of SEQ ID NO: 3, miR159 of SEQ ID NO: 4, miR162 of SEQ ID NO: 5, miR166 of SEQ ID NO: 6, miR167 of SEQ ID NO: 7, miR168 of SEQ ID NO: 8, miR169 of SEQ ID NO: 9, miR171 of SEQ ID NO: 10, miR172 of SEQ ID NO: 11, miR319 of SEQ ID NO: 12, miR393 of SEQ ID NO: 13, miR395 of SEQ ID NO: 14, miR396 of SEQ ID NO: 15, miR397 of SEQ ID NO: 16, miR398 of SEQ ID NO: 17, miR408 of SEQ ID NO: 18, miR477 of SEQ ID NO: 19, miR482a of SEQ ID NO: 20, miR482b of SEQ ID NO: 21, miR827 of SEQ ID NO: 22, miR1311 of SEQ ID NO: 23, miR1511 of SEQ ID NO: 24, miR1515 of SEQ ID NO: 25, miR3701 of SEQ ID NO: 26, miR4407 of SEQ ID NO: 27, miR4415 of SEQ ID NO: 28, miR6024 of SEQ ID NO: 29, miR6300 of SEQ ID NO: 30, gfc-lal-miR001 of SEQ ID NO: 31, gfc-lal-miR002 of SEQ ID NO: 32, gfc-lal-miR003 of SEQ ID NO: 33, gfc-lal-miR004 of SEQ ID NO: 34, gfc-lal-miR005 of SEQ ID NO: 35, gfc-cas-miR001 of SEQ ID NO: 36, gfc-cas-miR002 of SEQ ID NO: 37, gfc-cas-miR003 of SEQ ID NO: 38, gfc-cas-miR004 of SEQ ID NO: 39, and gfc-pgi-miR001 of SEQ ID NO: 40, and a skin improvement method using the same.

In order to predict whether the miRNA directly targets and regulates the expression of human genes which are heterologous, miRNA targets were predicted from the miRDB site (https://mirdb.org/). As a result, genes related to wound healing, skin moisturizing, whitening, elasticity, regeneration, wrinkle reduction, anti-aging, skin soothing, and inflammation were targeted, and predicted to have a skin improvement effect.

According to one embodiment of the present invention, the skin improvement may be at least one selected from the group consisting of skin moisturizing, whitening, elasticity, regeneration, wrinkle reduction, anti-aging, skin soothing, and anti-inflammation.

In addition, the present invention relates to a pharmaceutical composition for skin improvement containing, as an active ingredient, plant callus-derived exosomes containing at least one miRNA selected from the group consisting of miR156 of SEQ ID NO: 1, miR156b of SEQ ID NO: 2, miR157 of SEQ ID NO: 3, miR159 of SEQ ID NO: 4, miR162 of SEQ ID NO: 5, miR166 of SEQ ID NO: 6, miR167 of SEQ ID NO: 7, miR168 of SEQ ID NO: 8, miR169 of SEQ ID NO: 9, miR171 of SEQ ID NO: 10, miR172 of SEQ ID NO: 11, miR319 of SEQ ID NO: 12, miR393 of SEQ ID NO: 13, miR395 of SEQ ID NO: 14, miR396 of SEQ ID NO: 15, miR397 of SEQ ID NO: 16, miR398 of SEQ ID NO: 17, miR408 of SEQ ID NO: 18, miR477 of SEQ ID NO: 19, miR482a of SEQ ID NO: 20, miR482b of SEQ ID NO: 21, miR827 of SEQ ID NO: 22, miR1311 of SEQ ID NO: 23, miR1511 of SEQ ID NO: 24, miR1515 of SEQ ID NO: 25, miR3701 of SEQ ID NO: 26, miR4407 of SEQ ID NO: 27, miR4415 of SEQ ID NO: 28, miR6024 of SEQ ID NO: 29, miR6300 of SEQ ID NO: 30, gfc-lal-miR001 of SEQ ID NO: 31, gfc-lal-miR002 of SEQ ID NO: 32, gfc-lal-miR003 of SEQ ID NO: 33, gfc-lal-miR004 of SEQ ID NO: 34, gfc-lal-miR005 of SEQ ID NO: 35, gfc-cas-miR001 of SEQ ID NO: 36, gfc-cas-miR002 of SEQ ID NO: 37, gfc-cas-miR003 of SEQ ID NO: 38, gfc-cas-miR004 of SEQ ID NO: 39, and gfc-pgi-miR001 of SEQ ID NO: 40, and a skin improvement/treatment method using the same.

In addition, the present invention relates to a filler composition for skin improvement containing, as an active ingredient, plant callus-derived exosomes containing at least one miRNA selected from the group consisting of miR156 of SEQ ID NO: 1, miR156b of SEQ ID NO: 2, miR157 of SEQ ID NO: 3, miR159 of SEQ ID NO: 4, miR162 of SEQ ID NO: 5, miR166 of SEQ ID NO: 6, miR167 of SEQ ID NO: 7, miR168 of SEQ ID NO: 8, miR169 of SEQ ID NO: 9, miR171 of SEQ ID NO: 10, miR172 of SEQ ID NO: 11, miR319 of SEQ ID NO: 12, miR393 of SEQ ID NO: 13, miR395 of SEQ ID NO: 14, miR396 of SEQ ID NO: 15, miR397 of SEQ ID NO: 16, miR398 of SEQ ID NO: 17, miR408 of SEQ ID NO: 18, miR477 of SEQ ID NO: 19, miR482a of SEQ ID NO: 20, miR482b of SEQ ID NO: 21, miR827 of SEQ ID NO: 22, miR1311 of SEQ ID NO: 23, miR1511 of SEQ ID NO: 24, miR1515 of SEQ ID NO: 25, miR3701 of SEQ ID NO: 26, miR4407 of SEQ ID NO: 27, miR4415 of SEQ ID NO: 28, miR6024 of SEQ ID NO: 29, miR6300 of SEQ ID NO: 30, gfc-lal-miR001 of SEQ ID NO: 31, gfc-lal-miR002 of SEQ ID NO: 32, gfc-lal-miR003 of SEQ ID NO: 33, gfc-lal-miR004 of SEQ ID NO: 34, gfc-lal-miR005 of SEQ ID NO: 35, gfc-cas-miR001 of SEQ ID NO: 36, gfc-cas-miR002 of SEQ ID NO: 37, gfc-cas-miR003 of SEQ ID NO: 38, gfc-cas-miR004 of SEQ ID NO: 39, and gfc-pgi-miR001 of SEQ ID NO: 40, and a skin improvement method using the same.

Preferably, the cosmetic composition, pharmaceutical composition and filler composition for skin improvement according to the present invention may contain at least one selected from SEQ ID NO: 1, SEQ ID NO: 15, SEQ ID NO: 18, SEQ ID NO: 31, SEQ ID NO: 36 and SEQ ID NO: 40. More preferably, the cosmetic composition, pharmaceutical composition and filler composition for skin improvement according to the present invention may contain at least one selected from SEQ ID NO: 1, SEQ ID NO: 15 and SEQ ID NO: 18.

In one embodiment, the cosmetic composition, pharmaceutical composition and filler composition for skin improvement according to the present invention may be used for cosmetics, pharmaceuticals or quasi-drugs, and thus may be used for skin moisturizing, whitening, elasticity, regeneration, wrinkle reduction, anti-aging, skin soothing and anti-inflammation.

In addition, the cosmetic composition, pharmaceutical composition and filler composition for skin improvement according to the present invention may further contain pharmaceutically or cosmetically acceptable ingredients.

When the cosmetic composition, pharmaceutical composition and filler composition for skin improvement according to the present invention are used for cosmetics, the ingredients contained in conventional cosmetics include, but are not limited to, purified water, oil, wax, fatty acid, fatty acid alcohol, fatty acid ester, a surfactant, a humectant, a thickening agent, an antioxidant, a viscosity stabilizer, a chelating agent, buffer, and a lower alcohol, as carriers, excipients or diluents.

In addition, the cosmetic formulation including the cosmetic composition, pharmaceutical composition and filler composition for skin improvement according to the present invention may be prepared as any formulation commonly prepared in the art, and may be in the form of a solution, emulsion, viscous mixture, etc. Preferably, it may be one formulation selected from the group consisting of paste, emulsion, cream, gel, powder, spray, solution, emulsion, suspension, pack, foundation, lotion, cosmetic water, and surfactant-containing cleansing formulations. More specifically, the cosmetic composition includes one formulation selected from the group consisting of skin lotion, skin softener, skin toner, astringent, lotion, milk lotion, moisture lotion, nutritional lotion, cream, massage cream, nutritional cream, moisture cream, hand cream, foundation, essence, nutritional essence, pack, soap, cleansing foam, cleansing lotion, cleansing cream, body lotion, and body cleanser.

In addition, when the composition of the present invention is used for pharmaceuticals, it may be used in the form of a solid, semi-solid, solution, emulsion, dispersion, micelle, liposome, etc.

According to one embodiment of the present invention, the cosmetic composition, pharmaceutical composition and filler composition for skin improvement may be any one formulation selected from the group consisting of liquid, gel, solid, patch, injection, infusion, and spray formulations.

Although the preferred dosage of the cosmetic composition, pharmaceutical composition and filler composition for skin improvement according to the present invention varies depending on the condition and weight of the subject (patient), the severity of the disease, the form of the drug, and the route and period of administration, it may be appropriately selected by those skilled in the art.

The dosage of the cosmetic composition, pharmaceutical composition and filler composition for skin improvement according to the present invention may be appropriately selected by those skilled in the art, taking into consideration the mode of administration, the age, sex, and weight of the recipient, the severity of the disease, etc. For example, the dosage of the cosmetic/pharmaceutical composition of the present invention may be 0.001 to 1,000 mg/kg for adults. It may be administered once or several times a day, without being limited thereto.

### Mode for Invention

Hereinafter, the present invention will be described in more detail through examples. These examples are only intended to explain the present invention in more detail, and it will be apparent to those skilled in the art to which the present invention pertains that the scope of the present invention according to the subject matter of the present invention is not limited by these examples.

### <Example 1> Preparation of plant-derived callus and exosomes

### 1-1. Isolation of Leontopodium alpinum-derived callus culture (La-C) and exosomes (La-EV)

*Leontopodium alpinum* seeds (purchased from Chilternseeds, UK) were soaked in 70% ethanol for 1 minute to remove contaminants, then soaked in a 50% bleach solution containing 0.01% Tween-20 as a surfactant for 20 minutes, and then washed at least three times with sterile distilled water. The washed seeds were dried with sterilized filter paper to remove surface moisture, and then sown on MS (Murashige and Skoog, 1952) solid medium containing 30 g/L sucrose and 4 g/L gelatin without the addition of hormones for germination, and then induced to germinate at 25°C+2°C for one week under light conditions (4,000 lux). After germination, growth was maintained under light/dark conditions (16 hr/8 hr). Plant leaves aged 2 to 3 months after germination were cut to a size of 4*4 mm using a sterilized scalpel, and then cultured in the dark on MS medium containing 0.5 mg/L of the cytokinin hormone 6-benzylaminopurine (6-BA) and 0.3 mg/L of the auxin hormone 2,4-dichlorophenoxyacetic acid (2,4-D) for callus induction. After callus induction was confirmed microscopically in each explant, subculture was performed 3 or 4 times at 4-week intervals to select superior cell lines. After selecting the superior cell lines, the *in vitro-introduced Leontopodium alpinum* (La) callus culture was subjected to liquid suspension culture (3D CALLUSPHERE^{™}) for exosome extraction. Liquid suspension culture (3D CALLUSPHERE^{™}) is a plant cell suspension culture technology. It is a sterile 3D callus culture system in which cells may be cultured smoothly in suspension without stagnation by injecting air at an appropriate flow rate through an air pump in a bioreactor, a cell culture vessel made of glass, and the gas accumulated in the culture vessel through respiration may be discharged through the exhaust port. The culture medium used was MS liquid medium containing 0.5 mg/L 6-BA (6-benzylaminopurine) and 0.3 mg/L 2,4-D (2,4-dichlorophenoxyacetic acid) as plant growth hormones for *Leontopodium alpinum* callus growth and 3% sucrose as a carbon source. While the supply amount of oxygen-containing air using an air pump was maintained at 0.2 to 0.4 vvm, the culture was performed under a temperature condition of 23±2°C for about 6 weeks. The *Leontopodium alpinum* callus suspension culture cultured through 3D CALLUSPHERE^{™} was separated into the callus culture and the culture medium through a 200-mesh sieve. Callus-derived small RNA was extracted from the callus culture (La-C). Meanwhile, to prepare *Leontopodium alpinum* culture-derived exosomes (La-EV), the *Leontopodium alpinum* callus culture medium obtained through the 200-mesh sieve was subjected to physical stimulation of the cell membrane through low-frequency ultrasound treatment at 20 to 25 kHz for about 30 minutes. Next, after centrifugation at 1,500 to 2,000xg for 20 to 30 minutes using a centrifuge, only the supernatant was collected, thereby separating and removing cell debris. The supernatant extract was then subjected to a Hybrid-Exotech^{™} process for pure exosome extraction and concentration. The Hybrid-Exotech^{™} process was performed by a three-step process. Here, the first step was tangential flow filtration (TFF) using a 300-kDa annular porous membrane filter. In this step, the material was dispersed and circulated using continuous hydraulic pressure, thereby concentrating the material to about 5 to 10 times the initial starting volume based on particle size or molecular weight and performing first purification. In the second step, the exosomes concentrated and purified by TFF were subjected to second purification using ultracentrifugation at 100,000 to 150,000xg for 90 min. Finally, in the third step, third purification of collecting only pure nano-sized exosomes from which non-extracellular vesicles and large proteins have been removed was performed through bind-elute size-exclusion chromatography (BE-SEC) according to molecular size and molecular weight. The *Leontopodium alpinum* callus culture-derived exosomes obtained through the three-step Hybrid-Exotech^{™} process were filtered through a 0.22-µm filter, thereby preparing the final *Leontopodium alpinum* callus-derived exosomes (Ed-EV) that have been subjected to the sterilization process.

### 1-2. Isolation of Centella asiatica-derived callus culture (Ca-C) and exosomes (Ca-EV)

For *in vitro* introduction of *Centella asiatica,* fresh *Centella asiatica* (Seoul Floriculture) was purchased, the leaves were soaked in 95% ethanol for 1 minute, disinfected with a disinfectant solution (50% bleach + 0.1% Tween-20) for 20 minutes, and then washed 3 or 4 times with sterilized distilled water. The disinfected *Centella asiatica* leaves were cut into explants having a size of 4*4 mm (length*width) using a sharp knife and cultured in the dark at a temperature of 23+2°C on MS solid medium containing 1.0 mg/L of 6-benzylaminopurine (6-BA), 0.3 mg/L of 2,4-D (2,4-dichlorophenoxyacetic acid), 3% sucrose, and 4 g/L of gelatin to induce callus. After callus induction, a superior cell line was selected through subculture at 4-week intervals. The *Centella asiatica* (Ca) callus culture of the selected superior cell line was subjected to liquid suspension culture (3D CALLUSPHERE^{™}) in liquid medium from which gelatin had been removed. Liquid suspension culture (3D CALLUSPHERE^{™}) is a plant cell suspension culture technology. It is a sterile 3D callus culture system in which cells may be cultured smoothly in suspension without stagnation by injecting air at an appropriate flow rate through an air pump in a bioreactor, a cell culture vessel made of glass, and the gas accumulated in the culture vessel through respiration may be discharged through the exhaust port. The culture medium used was MS liquid medium containing 1.0 mg/L 6-BA (6-benzylaminopurine) and 0.3 mg/L 2,4-D (2,4-dichlorophenoxyacetic acid) as plant growth hormones for *Centella asiatica* callus growth and 3% sucrose as a carbon source. While the supply of oxygen-containing air using an air pump was maintained at 0.2 to 0.4 vvm, the culture was performed under a temperature condition of 23±2°C for about 6 weeks. The *Centella asiatica* callus suspension culture cultured through 3D CALLUSPHERE^{™} was separated into the callus culture and the culture medium through a 200-mesh sieve. Callus-derived small RNA was extracted from the callus culture (Ca-C). Meanwhile, to prepare *Centella asiatica* culture-derived exosomes (Ca-EV), the *Centella asiatica* callus culture medium obtained through the 200-mesh sieve was subjected to physical stimulation of the cell membrane through low-frequency ultrasound treatment at 20 to 25 kHz for about 30 minutes. Next, after centrifugation at 1,500 to 2,000xg for 20 to 30 minutes using a centrifuge, only the supernatant was collected, thereby separating and removing cell debris. The supernatant extract was then subjected to a Hybrid-Exotech^{™} process for pure exosome extraction and concentration. The Hybrid-Exotech^{™} process was performed by a three-step process. Here, the first step was tangential flow filtration (TFF) using a 300-kDa annular porous membrane filter. In this step, the material was dispersed and circulated using continuous hydraulic pressure, thereby concentrating the material to about 5 to 10 times the initial starting volume based on particle size or molecular weight and performing first purification. In the second step, the exosomes concentrated and purified by TFF were subjected to second purification using ultracentrifugation at 100,000 to 150,000xg for 90 min. Finally, in the third step, third purification of collecting only pure nano-sized exosomes from which non-extracellular vesicles and large proteins have been removed was performed through bind-elute size-exclusion chromatography (BE-SEC) according to molecular size and molecular weight. The *Centella asiatica* callus culture-derived exosomes obtained through the three-step Hybrid-Exotech^{™} process were filtered through a 0.22-µm filter, thereby preparing the final *Centella asiatica* callus-derived exosomes (Ca-EV) that have been subjected to the sterilization process.

### 1-3. Isolation of Panax ginseng-derived callus culture (Pg-C) and exosomes (Pg-EV)

For in vitro introduction of *Panax ginseng* (Pg), callus was induced using the roots of purchased *Panax ginseng* (Yakryong Market, Seoul). The purchased *Panax ginseng* roots were thoroughly washed in running water and then soaked in 95% ethanol for 1 minute. Next, the *Panax ginseng* roots were disinfected with a disinfectant solution (50% bleach + 0.1% Tween-20) for 20 minutes, and then washed 3 or 4 times with sterilized distilled water. The disinfected *Panax ginseng* roots were cut into explants having a size of 4*4 mm (length*width) using a sharp knife and plated on a solid medium containing 2 mg/L of growth hormone IBA (indole-butyric acid), 3% sucrose, and 4 g/L of gelatin. After callus induction under dark conditions, subculture was performed at 4-week intervals to select a superior cell line. The selected *Panax ginseng* culture was subjected to liquid suspension culture (3D CALLUSPHERE^{™}) for exosome extraction. Liquid suspension culture (3D CALLUSPHERE^{™}) is a plant cell suspension culture technology. It is a sterile 3D callus culture system in which cells may be cultured smoothly in suspension without stagnation by injecting air at an appropriate flow rate through an air pump in a bioreactor, a cell culture vessel made of glass, and the gas accumulated in the culture vessel through respiration may be discharged through the exhaust port. The culture medium used was MS liquid medium containing 2 mg/L IBA (indole-butyric acid) as an auxin plant growth hormone for *Panax ginseng* callus growth and 3% sucrose as a carbon source. While the supply of oxygen-containing air using an air pump was maintained at 0.2 to 0.4 vvm, the culture was performed under a temperature condition of 23±2°C for about 6 weeks. The *Panax ginseng* callus suspension culture cultured through 3D CALLUSPHERE^{™} was separated into the callus culture and the culture medium through a 200-mesh sieve. Callus-derived small RNA was extracted from the callus culture (Pg-C). Meanwhile, to prepare *Panax ginseng* culture-derived exosomes (Pg-EV), the *Panax ginseng* callus culture medium obtained through the 200-mesh sieve was subjected to physical stimulation of the cell membrane through low-frequency ultrasound treatment at 20 to 25 kHz for about 30 minutes. Next, after centrifugation at 1,500 to 2,000xg for 20 to 30 minutes using a centrifuge, only the supernatant was collected, thereby separating and removing cell debris. The supernatant extract was then subjected to a Hybrid-Exotech^{™} process for pure exosome extraction and concentration. The Hybrid-Exotech^{™} process was performed by a three-step process. Here, the first step was tangential flow filtration (TFF) using a 300-kDa annular porous membrane filter. In this step, the material was dispersed and circulated using continuous hydraulic pressure, thereby concentrating the material to about 5 to 10 times the initial starting volume based on particle size or molecular weight and performing first purification. In the second step, the exosomes concentrated and purified by TFF were subjected to second purification using ultracentrifugation at 100,000 to 150,000xg for 90 min. Finally, in the third step, third purification of collecting only pure nano-sized exosomes from which non-extracellular vesicles and large proteins have been removed was performed through bind-elute size-exclusion chromatography (BE-SEC) according to molecular size and molecular weight. The *Panax ginseng* callus culture-derived exosomes obtained through the three-step Hybrid-Exotech^{™} process were filtered through a 0.22-µm filter, thereby preparing the final *Panax ginseng* callus-derived exosomes (Pg-EV) that have been subjected to the sterilization process.

### <Experimental Example 1> Analysis of plant callus-derived exosomes

The particle size and concentration of the plant callus-derived exosomes obtained in Example 1 were measured using a nanoparticle tracking analyzer (ZetaView TWIN, Particle Metrix, Germany).

**[Table 1]**

| Sample name | Particle size (nm) | Concentration (particles/mL) |
|---|---|---|
| *Leontopodium alpinum* callus-derived exosomes (La-EV) | 141.3 | 1.3x10¹¹ |
| *Centella asiatica* callus-derived exosomes (Ca-EV) | 133.4 | 1.3x10¹⁰ |
| *Panax ginseng* callus-derived exosomes (Pg-EV) | 116.1 | 2.8x10¹⁰ |

As a result, as shown in Table 1 above and FIGS. 3a to 3f, it was confirmed that the particle sizes were 141.3 nm for the *Leontopodium alpinum* callus-derived exosomes, 133.4 nm for the *Centella asiatica* callus-derived exosomes, and 116.1 nm for the *Panax ginseng* callus-derived exosomes, which were all a nano-size of 150 nm or less.

Meanwhile, bicinchoninic acid (BCA) assay was performed for protein quantification. 20 µl of each plant callus-derived exosome sample obtained in Example 1 and 180 µl of the BCA sample were mixed together in a 96-well plate, and then incubated at 37°C for 1 hour while blocking light with foil. Thereafter, the absorbance was measured at a wavelength of 562 nm using a microplate reader (BioTek). The concentration of protein through the measured absorbance was analyzed through a standard curve of absorbance using bovine serum albumin (BSA), a standard protein substance provided along with the reagent. The protein concentration in each exosome sample was measured after adjusting the particle number to 10 billion (1.0x10¹⁰ particles/mL). The XENO-EVARI kit (Xenohelix, Korea) was used to extract small RNA from exosomes.

**[Table 2]**

| Sample name | Protein concentration (µg/1.0x10¹⁰ particles/mL) | Small RNA concentration (ng/µl) |
|---|---|---|
| *Leontopodium alpinum* callus-derived exosomes (La-EV) | 1,500 | 50.1 |
| *Centella asiatica* callus-derived exosomes (Ca-EV) | 1,700 | 45.8 |
| *Panax ginseng* callus-derived exosomes (Pg-EV) | 1,300 | 43.3 |

As a result, as shown in Table 2 above, the protein concentration in 10 billion exosomes (1.0x10¹⁰ particles/mL) was 1,700 µg in the *Centella asiatica* callus-derived exosomes (Ca-EV), 1, 500 µg in the *Leontopodium alpinum* callus-derived exosomes (La-EV), and 1,300 µg in the *Panax ginseng* callus-derived exosomes (Pg-EV), and the protein content in the exosomes was detected to be the highest in the *Centella asiatica* callus-derived exosomes (Ca-EV). In addition, as a result of extracting small RNA for miRNA analysis from each sample, it could be confirmed that the small RNA concentration was generally 43 to 50 ng/µl. After analyzing the purity of small RNA with a Bioanalyzer (Agilent 2100, USA), the small RNA was used as a sample for small RNA sequence analysis.

### <Experimental Example 2> Extraction and sequence analysis of small RNA from plant tissue culture callus and exosomes

Small RNAs were extracted from the *Leontopodium alpinum* callus (La-C) and exosomes (La-EV), *Centella asiatica* callus (Ca-C) and exosomes (Ca-EV), and *Panax ginseng* callus (Pg-C) and exosomes (Pg-EV) obtained in Example 1. Small RNA extraction from the plant callus samples was performed using a XENO-Plant small RNA Purification kit (Xenohelix, Korea), and small RNA extraction from the exosomes was performed using a XENO-EVARI kit (Xenohelix, Korea). NGS library construction and analysis for small RNA analysis were performed. Using a XENO-Libera library kit (Xenohelix, Korea), 3' and 5' adapters were ligated to both ends, and cDNA was generated by reverse transcription, and then amplified. The constructed library was sequenced using the MiniSeq sequencing system (Illumina). Raw data were checked for quality using FastQC v0.11. 9, and adapter sequences and low-quality 3'-end sequences were removed (quality score < 22) using Cutadapt v 2.8, and the read length was trimmed to 18 to 30 bases. The identified miRNAs were validated by calculating the number of aligned reads for each sample and the RPTM (Reads Per Ten Million) value.

**[Table 3]**

| **SEQ ID NO.** | **miRNA** | **Expressing plant** | **Sequence** | **Length (nt)** |
|---|---|---|---|---|
| SEQ ID NO: 1 | miR156 | Common | UGACAGAAGAGAGUGAGCACC | 21 |
| SEQ ID NO: 2 | miR156b | *Centella asiatica, Panax ginseng* | UGACAGAAGAGAGUGAGCACA | 21 |
| SEQ ID NO: 3 | miR157 | *Leontopodium alpinum* | UUGACAGAAGAUAGAGAGCAC | 21 |
| SEQ ID NO: 4 | miR159 | *Leontopodium alpinum* | UUGGACUGAAGGGAGCUCCC | 20 |
| SEQ ID NO: 5 | miR162 | *Leontopodium alpinum* | CUCAACUUCAAGCAAAGCUU | 20 |
| SEQ ID NO: 6 | miR166 | *Leontopodium alpinum, Centella asiatica* | UCGGACCAGGCUUCAUUCCCC | 21 |
| SEQ ID NO: 7 | miR167 | *Centella asiatica* | UCAGGUCAUCUUGCAGCUUCA | 21 |
| SEQ ID NO: 8 | miR168 | *Centella asiatica, Panax ginseng* | UCGCUUGGUGCAGGUCGGGAC | 21 |
| SEQ ID NO: 9 | miR169 | *Leontopodium alpinum* | UGUAAUCCUUAGCUUUGUGGUU | 22 |
| SEQ ID NO: 10 | miR171 | *Leontopodium alpinum, Centella asiatica* | UGAUUGAGCCGUGCCAAUAUC | 21 |
| SEQ ID NO: 11 | miR172 | *Centella asiatica* | AGAUGAUUUAUAUGUUACUG | 20 |
| SEQ ID NO: 12 | miR319 | *Leontopodium alpinum* | GCUGCUUUACAAUGUCUCUAU | 21 |
| SEQ ID NO: 13 | miR393 | *Centella asiatica* | UCCAAAGGGAUCGCAUUGAUC | 21 |
| SEQ ID NO: 14 | miR395 | *Centella asiatica* | CUGAAGUGUUUGGGGGAACU | 20 |
| SEQ ID NO: 15 | miR396 | Common | UUCCACAGCUUUCUUGAACU | 20 |
| SEQ ID NO: 16 | miR397 | *Leontopodium alpinum, Centella asiatica* | UCAUUGAGUGCAGCGUUGAUG | 21 |
| SEQ ID NO: 17 | miR398 | *Leontopodium alpinum, Panax ginseng* | CAUGUGUUCUCAUGUCACACC | 21 |
| SEQ ID NO: 18 | miR408 | Common | UUCCACAGCUUUCUUGAACU | 20 |
| SEQ ID NO: 19 | miR477 | *Leontopodium alpinum* | AUCUCCCUCAAAGGCUUCCAA | 21 |
| SEQ ID NO: 20 | miR482a | *Centella asiatica* | GGUGUGGCGUCUCUGGGGAGG | 21 |
| SEQ ID NO: 21 | miR482b | *Centella asiatica* | UUUCCUAUUCCACCCAUCCCAU | 22 |
| SEQ ID NO: 22 | miR827 | *Centella asiatica, Panax ginseng* | UUAGAUGACCAUCAACAAACU | 21 |
| SEQ ID NO: 23 | miR1311 | *Leontopodium alpinum* | UCAGAGUUUUGCCAGUUCCGCC | 22 |
| SEQ ID NO: 24 | miR1511 | *Leontopodium alpinum* | AACCAGGCUCUGAUACCAUG | 20 |
| SEQ ID NO: 25 | miR1515 | *Centella asiatica* | UCAUUUUUGCGUGCAAUGAUCC | 22 |
| SEQ ID NO: 26 | miR3701 | *Leontopodium alpinum* | UAAACAGUGCCCACCCUUCAUC | 22 |
| SEQ ID NO: 27 | miR4407 | *Leontopodium alpinum* | CAGAGGAAGCAGCACUUGUACC | 22 |
| SEQ ID NO: 28 | miR4415 | *Leontopodium alpinum* | AAGUUGUGAUGAGAAUCAAUG | 21 |
| SEQ ID NO: 29 | miR6024 | *Leontopodium alpinum* | UUUUAGCAAGAGUUGUUUUACC | 22 |
| SEQ ID NO: 30 | miR6300 | *Leontopodium alpinum* | GUCGUUGUAGUAUAGUGG | 18 |
| SEQ ID NO: 31 | gfc-lal-miR001 | *Leontopodium alpinum* | AACAGGAAUUUCGGCUCUGAUA | 22 |
| SEQ ID NO: 32 | gfc-lal-miR002 | *Leontopodium alpinum* | AGGGAGCUCCCUUCAGUCCAA | 21 |
| SEQ ID NO: 33 | gfc-lal-miR003 | *Leontopodium alpinum* | GUUGAAGAGUCAUUGUGGCCC | 21 |
| SEQ ID NO: 34 | gfc-lal-miR004 | *Leontopodium alpinum* | UCGGUUUUGAUUAUUCUACU | 20 |
| SEQ ID NO: 35 | gfc-lal-miR005 | *Leontopodium alpinum* | UCUAUGGGUUUGUUGAUUGCC | 21 |
| SEQ ID NO: 36 | gfc-cas-miR001 | *Centella asiatica* | GGUGUGGCGUCUCUGGGGAGG | 21 |
| SEQ ID NO: 37 | gfc-cas-miR002 | *Centella asiatica* | UUGGUCGGUGGUCUAUGUCACA | 22 |
| SEQ ID NO: 38 | gfc-cas-miR003 | *Centella asiatica* | UCUGAAAGAUUGUUUGUGGGG | 21 |
| SEQ ID NO: 39 | gfc-cas-miR004 | *Centella asiatica* | UAGAAAACUCAUAAUCGACU | 20 |
| SEQ ID NO: 40 | gfc-pgi-miR001 | *Panax ginseng* | CUGCGUCGCUCCGAUUCGUC | 20 |

As a result, as shown in FIGS. 4 to 6, the entire sequencing results were classified into Chloroplast, CDS, tRNA, rRNA, ncRNA, and miRNA precursor based on the genomic sequence database or miRNA database of each plant, and those with unknown functions were classified as "Unclassified". Referring to FIG. 4, about 50 to 60% of sequences in all the plants were classified as "unclassified" with unknown functions. In addition, CDS indicating gene functions were high in the *Panax ginseng* callus, *Centella asiatica* callus, and *Centella asiatica* callus-derived exosomes, and in the case of *Leontopodium alpinum,* which has almost no gene database, low values were shown in both the *Leontopodium alpinum* callus and the *Leontopodium alpinum* callus-derived exosomes. Validation of each miRNA was performed using the existing miRNA database, miRBase (www.mirbase.org). As a result, callus-derived miRNAs were found to be generally more numerous than exosome-derived miRNAs, and about 5 to 10% of these miRNAs were found to be present commonly in the exosomes. This differs between the plants, and in the case of *Leontopodium alpinum,* where the existing genome sequencing database is insufficient, the largest number of miRNAs was secured. That is, about 200 miRNAs based on callus were previously reported, and among them, 45 miRNAs could be identified in the exosomes. Among them, about 40 miRNAs with a sequence identity of 90% or more to previously reported ones and 5 novel miRNAs with no sequence identity were identified. In the case of *Centella asiatica,* about 70 callus-based miRNAs were identified, and among them, 26 previously reported miRNAs and 4 novel miRNAs were identified to be present in the exosomes. In the case of *Panax ginseng,* although the genome database was larger than those of *Leontopodium alpinum* and *Centella asiatica,* the number of miRNAs was the smallest (30), and among these miRNAs, only 8 miRNAs, including 1 novel miRNA, were identified to be present in the exosomes.

FIGS. 5a to 5c are graphs showing the top miRNA families in *Leontopodium alpinum* callus-derived exosomes, *Centella asiatica* callus-derived exosomes, and *Panax ginseng* callus-derived exosomes, identified through NGS-based small RNA analysis. These figures show the families of top 20 conserved miRNAs highly expressed in *Leontopodium alpinum* callus-derived exosomes and *Centella asiatica* callus-derived exosomes, while in the case of *Panax ginseng* callus-derived exosomes, the number of miRNAs is generally small, and thus only 7 miRNAs are shown.

### <Experimental Example 3> miRNA validation by qRT-PCR

To validate the expression levels of novel miRNAs specifically expressed in each plant within *Leontopodium alpinum* callus-derived exosomes (La-EV), *Centella asiatica* callus-derived exosomes (Ca-EV), and *Panax ginseng* callus-derived exosomes (Pg-EV), identified through NGS-based small RNA analysis, qRT-PCR was performed. For miRNA detection, stem-loop RT-PCR was used, in which SLP (Stem-Loop Reverse Primer) was designed to form a hairpin and have a 3' overhang complementary to the miRNA. A miRNA-specific forward primer with a 5' adapter and a universal reverse primer (URP) were designed for PCR amplification. Using the Mir-X miRNA qRT-PCR TB Green^{™} Kit, cDNA synthesis from miRNA and qRT-PCR using the synthesized cDNA were performed. The qRT-PCR reaction was performed under the following conditions: one cycle of 95°C for 10 min for denaturation, followed by 40 cycles of 95°C for 10 sec and 60°C for 30 sec for amplification. U6 snRNA as an internal normalization control was provided in the kit. Relative gene expression levels were calculated using the delta-delta Ct (ddCt) method. All reactions were repeated three times for each target.

As a result, as shown in FIGS. 7a and 7b, it was confirmed that miRNAs of *Leontopodium alpinum* callus-derived exosome (La-EV), *Centella asiatica* callus-derived exosome (Ca-EV), and *Panax ginseng* callus-derived exosome (Pg-EV) were expressed 2.0 to 3.5 times more specifically in the respective plants. Similarly, as a result of electrophoresing the PCR products on 2% agarose gel, it was confirmed visually that the expression of miRNAs increased compared to U6, which was used as a control.

### <Experimental Example 4> Evaluation of skin improvement effect of miRNAs derived from plant callus exosomes

The expression levels of exosome-specific miRNAs obtained through RNA sequencing of *Leontopodium alpinum* callus-derived exosomes, *Centella asiatica* callus-derived exosomes, and *Panax ginseng* callus-derived exosomes were analyzed. As a result, among the miRNAs, miR156 of SEQ ID NO: 1, miR396 of SEQ ID NO: 15, and miR408 of SEQ ID NO: 18 that are commonly expressed, gfc-lal-miR001 of SEQ ID NO: 31 that is specific to *Leontopodium alpinum* callus-derived exosomes, gfc-cas-miR001 of SEQ ID NO: 36 that is specific to *Centella asiatica* callus-derived exosomes, and gfc-pgi-miR001 of SEQ ID NO: 40 that is specific to *Panax ginseng* callus-derived exosomes were subjected to an experiment for evaluating their functions in human skin cells. miR156, miR396, miR408, gfc-lal-miR001, gfc-cas-miR001, gfc-pgi-miR001 mimic oligomers were synthesized, and microRNA mimic (IDT, USA), a double-stranded RNA oligonucleotide, was used as a control. Human epidermal keratinocytes (HaCaT) and human dermal fibroblasts (HDF) were used as cell lines for the experiment. To introduce and express miRNAs in each cell line, miR156, miR396, miR408, gfc-lal-miR001, gfc-cas-miR001, gfc-pgi-miR001, and microRNA mimic (miR-Con) were introduced into the cells at a concentration of 10 nM using lipofectamine (Invitrogen). After 48 hours, the change in cell viability (proliferation rate compared to miR-Con) was measured using WST-1 assay.

**[Table 4]**

| | **Cell proliferation rate (%)** | |
|---|---|---|
| | **HaCaT** | **HDF** |
| miR-Con | 100 | 100 |
| miR156 (SEQ ID NO: 1) | 120 | 125 |
| miR396 (SEQ ID NO: 15) | 132 | 132 |
| miR408 (SEQ ID NO: 18) | 129 | 129 |
| gfc-lal-miR001 (SEQ ID NO: 31) | 130 | 131 |
| gfc-cas-miR001 (SEQ ID NO: 36) | 119 | 125 |
| gfc-pgi-miR001 (SEQ ID NO: 40) | 128 | 130 |

As a result, as shown in Table 4 above, it was confirmed that transfection of the HaCaT and HDF cells with each miRNA at a concentration of 10 nM exhibited an increase in cell proliferation rate of about 20 to 30% compared to miR-Con (control) without toxicity.

In addition, after incubating the transfected cells in an incubator containing 5% CO₂ at 37°C for about 48 hours, RNA was isolated using 1 mL of Trizol reagent. Then, cDNA (C1000 Thermal Cycler, Bio-Rad, USA) was synthesized from the isolated RNA using oligo-dT and reverse transcriptase. To select target genes, analysis in the human genome was performed using the website miRDB, which can predict miRNA targets, and as a result, FLG (filaggrin), a moisturizing-related gene, COL1A1 (collagen type I alpha 1), a regeneration- and aging-related gene, and CER2 (ceramide synthase 2), a skin barrier-related gene were identified. Therefore, the expression level of each target gene was finally evaluated by performing a polymerase chain reaction in a real-time PCR machine using a mixture containing each target gene-specific primer and the cyanine dye CyberGreen (SYBR Green qPCR Master Mix, Applied Biosystems, USA). The sequences of the primers are shown in Table 5 below, and the expression level of each gene was finally analyzed after normalization to the β-actin gene.

**[Table 5]**

| | UniProt ID | Name | Direction | Sequence |
|---|---|---|---|---|
| SEQ ID NOs: 41 and 42 | P20930 | FLG | Forward | GCTGAAGGAACTTCTGGAAAAGG |
| | | | Reverse | GTTGTGGTCTATATCCAAGTGATC |
| SEQ ID NOs: 43 and 44 | Q96G23 | CER2 | Forward | GCCTTGCTCTTCCTCATCGTTC |
| | | | Reverse | TGCTTGCCACTGGTCAGGTAGA |
| SEQ ID NOs: 45 and 46 | P02452 | COL1A1 | Forward | GATTCCCTGGACCTAAAGGTGC |
| | | | Reverse | AGCCTCTCCATCTTTGCCAGCA |
| SEQ ID NOs: 47 and 48 | P60709 | β-actin (ACTB) | Forward | CACCATTGGCAATGAGCGGTTC |
| | | | Reverse | AGGTCTTTGCGGATGTCCACGT |

As a result, as shown in FIG. 8, it was confirmed that, when the cells were transfected with mimics of miR156 of SEQ ID NO. 1, miR396 of SEQ ID NO. 15, and miR408 of SEQ ID NO. 18, which are commonly expressed, and gfc-lal-miR001 of SEQ ID NO. 31 specific to *Leontopodium alpinum* callus-derived exosomes, gfc-cas-miR001 of SEQ ID NO. 36 specific to *Centella asiatica* callus-derived exosomes, and gfc-pgi-miR001 of SEQ ID NO. 40 specific to *Panax ginseng* callus-derived exosomes, which are miRNA sequences specific to the respective plants, the expression of FLG (filaggrin), COL1A1 (collagen type I alpha 1), and CER2 (ceramide synthase 2) increased 1.2 to 3.4-fold compared to when transfected with microRNA mimic (miR-Con), an oligonucleotide that does not overlap at all with the miRNA sequence of the present invention. That is, miR156 of SEQ ID NO: 1 according to the present invention showed an about 3-fold increase in the expression of CER2 and COL1A1, miR396 of SEQ ID NO: 15 showed a 2.1-fold increase in the expression of CER2, and miR408 of SEQ ID NO: 18 showed the highest increase (3.4-fold) in the expression rate of COL1A1. gfc-lal-miR001 of SEQ ID NO: 31 showed the same expression pattern as miR156 of SEQ ID NO: 1, with a 1.5-fold increase in the expression of FLG, but an about 3-fold increase in the expression of CER2 and COL1A1. gfc-cas-miR001 of SEQ ID NO: 36 and gfc-pgi-miR001 of SEQ ID NO: 40 showed an about 2 to 3-fold increase in the expression of FLG, CER2, and COL1A1. This suggests that an isolated miRNA nucleic acid molecule including the sequence of miR156 of SEQ ID NO: 1, miR396 of SEQ ID NO: 15, miR408 of SEQ ID NO: 18, gfc-lal-miR001 of SEQ ID NO: 31, gfc-cas-miR001 of SEQ ID NO: 36, gfc-pgi-miR001 of SEQ ID NO: 40 or an antisense nucleic acid molecule thereof according to the present invention can effectively regulate the expression of FLG, COL1A1, and CER2. In addition, it was confirmed that an isolated miRNA nucleic acid molecule including the sequence of miR156 of SEQ ID NO: 1, miR396 of SEQ ID NO: 15, miR408 of SEQ ID NO: 18, gfc-lal-miR001 of SEQ ID NO: 31, gfc-cas-miR001 of SEQ ID NO: 36, gfc-pgi-miR001 of SEQ ID NO: 40 or an antisense nucleic acid molecule thereof according to the present invention can effectively regulate the expression of one or more of FLG, COL1A1, and CER2.

Although the present invention has been described in detail with reference to specific features, it will be apparent to those skilled in the art that this description is only of a preferred embodiment thereof, and does not limit the scope of the present invention. Thus, the substantial scope of the present invention will be defined by the appended claims and equivalents thereto.

### Industrial Applicability

According to the present invention, miR156 of SEQ ID NO: 1, miR156b of SEQ ID NO: 2, miR157 of SEQ ID NO: 3, miR159 of SEQ ID NO: 4, miR162 of SEQ ID NO: 5, miR166 of SEQ ID NO: 6, miR167 of SEQ ID NO: 7, miR168 of SEQ ID NO: 8, miR169 of SEQ ID NO: 9, miR171 of SEQ ID NO: 10, miR172 of SEQ ID NO: 11, miR319 of SEQ ID NO: 12, miR393 of SEQ ID NO: 13, miR395 of SEQ ID NO: 14, miR396 of SEQ ID NO: 15, miR397 of SEQ ID NO: 16, miR398 of SEQ ID NO: 17, miR408 of SEQ ID NO: 18, miR477 of SEQ ID NO: 19, miR482a of SEQ ID NO: 20, miR482b of SEQ ID NO: 21, miR827 of SEQ ID NO: 22, miR1311 of SEQ ID NO: 23, miR1511 of SEQ ID NO: 24, miR1515 of SEQ ID NO: 25, miR3701 of SEQ ID NO: 26, miR4407 of SEQ ID NO: 27, miR4415 of SEQ ID NO: 28, miR6024 of SEQ ID NO: 29, miR6300 of SEQ ID NO: 30, gfc-lal-miR001 of SEQ ID NO: 31, gfc-lal-miR002 of SEQ ID NO: 32, gfc-lal-miR003 of SEQ ID NO: 33, gfc-lal-miR004 of SEQ ID NO: 34, gfc-lal-miR005 of SEQ ID NO: 35, gfc-cas-miR001 of SEQ ID NO: 36, gfc-cas-miR002 of SEQ ID NO: 37, gfc-cas-miR003 of SEQ ID NO: 38, gfc-cas-miR004 of SEQ ID NO: 39, and gfc-pgi-miR001 of SEQ ID NO: 40 may be used as biomarkers for validating plant callus-derived exosomes by identifying that they are expressed in the plant callus-derived exosomes.

## Claims

1. A biomarker for validating plant callus-derived exosomes, comprising at least one selected from the group consisting of miR156 of SEQ ID NO: 1, miR156b of SEQ ID NO: 2, miR157 of SEQ ID NO: 3, miR159 of SEQ ID NO: 4, miR162 of SEQ ID NO: 5, miR166 of SEQ ID NO: 6, miR167 of SEQ ID NO: 7, miR168 of SEQ ID NO: 8, miR169 of SEQ ID NO: 9, miR171 of SEQ ID NO: 10, miR172 of SEQ ID NO: 11, miR319 of SEQ ID NO: 12, miR393 of SEQ ID NO: 13, miR395 of SEQ ID NO: 14, miR396 of SEQ ID NO: 15, miR397 of SEQ ID NO: 16, miR398 of SEQ ID NO: 17, miR408 of SEQ ID NO: 18, miR477 of SEQ ID NO: 19, miR482a of SEQ ID NO: 20, miR482b of SEQ ID NO: 21, miR827 of SEQ ID NO: 22, miR1311 of SEQ ID NO: 23, miR1511 of SEQ ID NO: 24, miR1515 of SEQ ID NO: 25, miR3701 of SEQ ID NO: 26, miR4407 of SEQ ID NO: 27, miR4415 of SEQ ID NO: 28, miR6024 of SEQ ID NO: 29, miR6300 of SEQ ID NO: 30, gfc-lal-miR001 of SEQ ID NO: 31, gfc-lal-miR002 of SEQ ID NO: 32, gfc-lal-miR003 of SEQ ID NO: 33, gfc-lal-miR004 of SEQ ID NO: 34, gfc-lal-miR005 of SEQ ID NO: 35, gfc-cas-miR001 of SEQ ID NO: 36, gfc-cas-miR002 of SEQ ID NO: 37, gfc-cas-miR003 of SEQ ID NO: 38, gfc-cas-miR004 of SEQ ID NO: 39, and gfc-pgi-miR001 of SEQ ID NO: 40.

2. The biomarker of claim 1, comprising at least one selected from the group consisting of SEQ ID NO: 1, SEQ ID NO: 15, and SEQ ID NO: 18.

3. A method for isolating plant callus-derived exosomes, comprising:
a step of isolating exosomes from plant callus;
a step of extracting small RNA from the exosomes isolated from plant callus;
a step of analyzing a nucleotide sequence of the extracted small RNA to obtain sequence data of miRNA; and
a step of validating the isolated exosomes by identifying at least one miRNA selected from the group consisting of miR156 of SEQ ID NO: 1, miR156b of SEQ ID NO: 2, miR157 of SEQ ID NO: 3, miR159 of SEQ ID NO: 4, miR162 of SEQ ID NO: 5, miR166 of SEQ ID NO: 6, miR167 of SEQ ID NO: 7, miR168 of SEQ ID NO: 8, miR169 of SEQ ID NO: 9, miR171 of SEQ ID NO: 10, miR172 of SEQ ID NO: 11, miR319 of SEQ ID NO: 12, miR393 of SEQ ID NO: 13, miR395 of SEQ ID NO: 14, miR396 of SEQ ID NO: 15, miR397 of SEQ ID NO: 16, miR398 of SEQ ID NO: 17, miR408 of SEQ ID NO: 18, miR477 of SEQ ID NO: 19, miR482a of SEQ ID NO: 20, miR482b of SEQ ID NO: 21, miR827 of SEQ ID NO: 22, miR1311 of SEQ ID NO: 23, miR1511 of SEQ ID NO: 24, miR1515 of SEQ ID NO: 25, miR3701 of SEQ ID NO: 26, miR4407 of SEQ ID NO: 27, miR4415 of SEQ ID NO: 28, miR6024 of SEQ ID NO: 29, miR6300 of SEQ ID NO: 30, gfc-lal-miR001 of SEQ ID NO: 31, gfc-lal-miR002 of SEQ ID NO: 32, gfc-lal-miR003 of SEQ ID NO: 33, gfc-lal-miR004 of SEQ ID NO: 34, gfc-lal-miR005 of SEQ ID NO: 35, gfc-cas-miR001 of SEQ ID NO: 36, gfc-cas-miR002 of SEQ ID NO: 37, gfc-cas-miR003 of SEQ ID NO: 38, gfc-cas-miR004 of SEQ ID NO: 39, and gfc-pgi-miR001 of SEQ ID NO: 40, which is expressed in the exosomes isolated from plant callus, from the nucleotide sequence of the miRNA.

4. The method of claim 3, wherein the step of validating the isolated exosomes by identifying the miRNA comprises a step of validating the exosomes by identifying at least one miRNA selected from the group consisting of SEQ ID NO: 1, SEQ ID NO: 15, and SEQ ID NO: 18.

5. The method of claim 3, wherein the plant is at least one selected from the group consisting of *Asteraceae, Apiaceae, Rutaceae, Rosaceae, Vitaceae, Brassicaceae, Selaginellaceae, Oleaceae, Nelumbonaceae, Araliaceae, Lamiaceae, Hypericaceae, Pedaliaceae, Ginkgoaceae,* and *Piperaceae.*

6. The method of claim 4, comprising validating whether the exosomes are exosomes derived from *Leontopodium alpinum, Centella asiatica* or *Panax ginseng* callus.

7. The method of claim 2, wherein the analysis of the nucleotide sequence is performed by measurement using a next-generation sequencing (NGS) method.

8. A plant callus-derived exosome obtained by the method of any one of claims 2 to 7.

9. A cosmetic composition for skin improvement comprising at least one miRNA selected from the group consisting of miR156 of SEQ ID NO: 1, miR156b of SEQ ID NO: 2, miR157 of SEQ ID NO: 3, miR159 of SEQ ID NO: 4, miR162 of SEQ ID NO: 5, miR166 of SEQ ID NO: 6, miR167 of SEQ ID NO: 7, miR168 of SEQ ID NO: 8, miR169 of SEQ ID NO: 9, miR171 of SEQ ID NO: 10, miR172 of SEQ ID NO: 11, miR319 of SEQ ID NO: 12, miR393 of SEQ ID NO: 13, miR395 of SEQ ID NO: 14, miR396 of SEQ ID NO: 15, miR397 of SEQ ID NO: 16, miR398 of SEQ ID NO: 17, miR408 of SEQ ID NO: 18, miR477 of SEQ ID NO: 19, miR482a of SEQ ID NO: 20, miR482b of SEQ ID NO: 21, miR827 of SEQ ID NO: 22, miR1311 of SEQ ID NO: 23, miR1511 of SEQ ID NO: 24, miR1515 of SEQ ID NO: 25, miR3701 of SEQ ID NO: 26, miR4407 of SEQ ID NO: 27, miR4415 of SEQ ID NO: 28, miR6024 of SEQ ID NO: 29, miR6300 of SEQ ID NO: 30, gfc-lal-miR001 of SEQ ID NO: 31, gfc-lal-miR002 of SEQ ID NO: 32, gfc-lal-miR003 of SEQ ID NO: 33, gfc-lal-miR004 of SEQ ID NO: 34, gfc-lal-miR005 of SEQ ID NO: 35, gfc-cas-miR001 of SEQ ID NO: 36, gfc-cas-miR002 of SEQ ID NO: 37, gfc-cas-miR003 of SEQ ID NO: 38, gfc-cas-miR004 of SEQ ID NO: 39, and gfc-pgi-miR001 of SEQ ID NO: 40.

10. The cosmetic composition of claim 9, wherein the skin improvement is at least one selected from the group consisting of skin moisturizing, whitening, elasticity, regeneration, wrinkle reduction, anti-aging, skin soothing, and anti-inflammation.

11. The cosmetic composition of claim 9, comprising at least one miRNA selected from SEQ ID NO: 1, SEQ ID NO: 15, SEQ ID NO: 18, SEQ ID NO: 31, SEQ ID NO: 36, and SEQ ID NO: 40.

12. A method for skin improvement comprising a step of administering to a subject a cosmetic composition comprising at least one miRNA selected from the group consisting of miR156 of SEQ ID NO: 1, miR156b of SEQ ID NO: 2, miR157 of SEQ ID NO: 3, miR159 of SEQ ID NO: 4, miR162 of SEQ ID NO: 5, miR166 of SEQ ID NO: 6, miR167 of SEQ ID NO: 7, miR168 of SEQ ID NO: 8, miR169 of SEQ ID NO: 9, miR171 of SEQ ID NO: 10, miR172 of SEQ ID NO: 11, miR319 of SEQ ID NO: 12, miR393 of SEQ ID NO: 13, miR395 of SEQ ID NO: 14, miR396 of SEQ ID NO: 15, miR397 of SEQ ID NO: 16, miR398 of SEQ ID NO: 17, miR408 of SEQ ID NO: 18, miR477 of SEQ ID NO: 19, miR482a of SEQ ID NO: 20, miR482b of SEQ ID NO: 21, miR827 of SEQ ID NO: 22, miR1311 of SEQ ID NO: 23, miR1511 of SEQ ID NO: 24, miR1515 of SEQ ID NO: 25, miR3701 of SEQ ID NO: 26, miR4407 of SEQ ID NO: 27, miR4415 of SEQ ID NO: 28, miR6024 of SEQ ID NO: 29, miR6300 of SEQ ID NO: 30, gfc-lal-miR001 of SEQ ID NO: 31, gfc-lal-miR002 of SEQ ID NO: 32, gfc-lal-miR003 of SEQ ID NO: 33, gfc-lal-miR004 of SEQ ID NO: 34, gfc-lal-miR005 of SEQ ID NO: 35, gfc-cas-miR001 of SEQ ID NO: 36, gfc-cas-miR002 of SEQ ID NO: 37, gfc-cas-miR003 of SEQ ID NO: 38, gfc-cas-miR004 of SEQ ID NO: 39, and gfc-pgi-miR001 of SEQ ID NO: 40.
